(19) Europäisches Patentamt
European Patent Office
Office européen des brevets

(11) Veröffentlichungsnummer: **0 236 838**
**A2**

(12) # EUROPÄISCHE PATENTANMELDUNG

(21) Anmeldenummer: **87102581.3**

(22) Anmeldetag: **24.02.87**

(51) Int.Cl.³: **C 07 D 211/90**
C 07 D 455/06, C 07 D 405/12
C 07 D 405/14, C 07 D 409/04
C 07 D 409/14, C 07 D 405/04
C 07 D 401/04, C 07 D 413/04
C 07 D 413/14, C 07 D 417/04

(30) Priorität: **08.03.86 DE 3607821**

(43) Veröffentlichungstag der Anmeldung:
**16.09.87 Patentblatt 87/38**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Schwenner, Eckhard, Dr.**
**Claudiusweg 3**
**D-5600 Wuppertal 1(DE)**

(72) Erfinder: **Stegelmeier, Hartmut, Dr.**
**Meide Id**
**D-4010 Hilden(DE)**

(72) Erfinder: **Kazda, Stanislav, Prof. Dr.**
**Gellertweg 18**
**D-5600 Wuppertal 1(DE)**

(72) Erfinder: **Knorr, Andreas, Dr.**
**Trillser Graben 10**
**D-4006 Erkrath 2(DE)**

(54) **Aminoester-Dihydropyridine, Verfahren zu ihrer Herstellung und ihre Verwendung.**

(57) Die Erfindung betrifft Aminoester-Dihydropyridine der allgemeinen Formel I

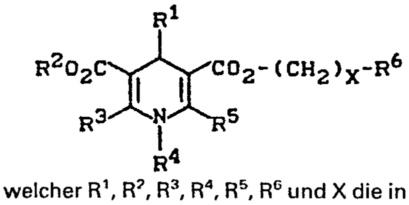

in welcher $R^1$, $R^2$, $R^3$, $R^4$, $R^5$, $R^6$ und X die in der Beschreibung angegebene Bedeutung haben, Verfahren zur Herstellung und ihre Verwendung als Arzneimittel, insbesondere als kreislaufbeeinflussende Arzneimittel.

EP 0 236 838 A2

0236838

BAYER AKTIENGESELLSCHAFT 5090 Leverkusen, Bayerwerk
Konzernverwaltung RP
Patentabteilung KS/bo/c

# Aminoester-Dihydropyridine, Verfahren zur Herstellung und ihre Verwendung

Die Erfindung betrifft Aminoester-Dihydropyridine, Verfahren zur Herstellung und ihre Verwendung als Arzneimittel, insbesondere als kreislaufbeeinflussende Arzneimittel.

Es ist bekannt, daß man 1,4-Dihydro-2,6-dimethyl-4-phenylpyridin-3,5-dicarbonsäurediethylester erhält, wenn man 2-Benzylidenacetessigsäureethylester mit β-Aminocrotonsäureester oder Acetessigsäureethylester mit Ammoniak umsetzt [E. Knoevenagel, Ber. dtsch. Chem. Ges. 31, 743 (1898)].

Außerdem ist bekannt, daß bestimmte 1,4-Dihydropyridine interessante pharmakologische Eigenschaften aufweisen [F. Bossert, W. Vater, Naturwissenschaften 58, 578 (1971)].

Die vorliegende Erfindung betrifft neue Aminoester-Dihydropyridine der allgemeinen Formel (I)

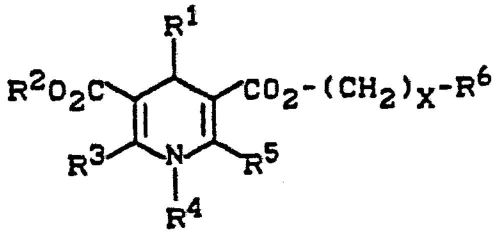

in welcher

$R^1$ - für $C_6$-$C_{14}$-Aryl oder für Heterocyclyl aus der Reihe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzothiadiazolyl, Chinolyl, Isochinolyl, Chinazolyl oder Chinoxalyl steht, wobei die genannten Ringsysteme jeweils durch ein oder zwei gleiche oder verschiedene Substituenten aus der Gruppe Phenyl, geradkettiges oder verzweigtes $C_1$-$C_4$-Alkyl, $C_3$-$C_7$-Cycloalkyl, $C_1$-$C_4$-Alkoxy, $C_1$-$C_4$-Alkylthio, Tri-, Tetra- oder Pentamethylen, Dioxymethylen, Dioxyethylen, Halogen, Trifluormethyl, Trifluormethoxy, Difluormethoxy, Tetrafluorethoxy, Nitro, Cyano oder Azido substituiert sein können,

$R^2$ - für einen geradkettigen, verzweigten oder cyclischen, gesättigten oder ungesättigen Kohlenwasserstoffrest mit bis zu 15 Kohlenstoffatomen steht, der gegebenenfalls durch ein Sauerstoffatom oder ein Schwefelatom in der Kette unterbrochen ist und/oder der gegebenenfalls

substituiert ist durch Halogen, Cyano, Hydroxy, $C_2$-$C_7$-Acyloxy, Nitro, Nitrooxy oder durch eine Phenyl-, Phenoxy-, Phenylthio- oder Phenylsulfonylgruppe, die ihrerseits gegebenenfalls durch Di-$C_1$-$C_2$-alkylamino, $C_1$-$C_4$-Alkoxy, $C_1$-$C_4$-Alkyl, Halogen, Trifluormethyl oder Nitro substituiert ist, oder durch eine α-, β- oder γ-Pyridylgruppe, oder durch eine Aminogruppe, wobei diese Aminogruppe zwei gleiche oder verschiedene Substituenten aus der Gruppe $C_1$-$C_4$-Alkyl, $C_1$-$C_6$-Alkoxyalkyl, Phenyl und Benzyl trägt oder wobei die Substituenten gegebenenfalls mit dem Stickstoffatom einen 5- bis 7-gliedrigen Ring bilden, der als weiteres Heteroatom ein Sauerstoff-oder Schwefelatom oder eine N-Phenyl- oder N-Alkylgruppierung enthalten kann, wobei die Alkylgruppe ein bis drei Kohlenstoffatome umfaßt,

$R^3$ und $R^5$ gleich oder verschieden sind und jeweils

- für Wasserstoff, einen geradkettigen, verzweigten oder cyclischen Alkylrest mit bis zu 8 Kohlenstoffatomen, einen Phenyl- oder Benzylrest stehen,

  oder

  einer der Substituenten $R^3$ oder $R^5$ einen Alkylrest mit bis zu 6 Kohlenstoffatomen darstellt, der durch Acetoxy, Benzoyloxy, $C_1$-$C_3$-Alkoxy, $C_1$-$C_3$-Dialkoxy, Hydroxy, Amino, $C_1$-$C_6$-Aminoalkoxy, Phthalimido, $C_1$-$C_6$-Phthalimidoalkoxy, $C_1$-$C_6$-Piperidinoalkoxy, $C_1$-$C_6$-Morpholinoalkoxy oder N-Phenyl-N'-piperazinoalkoxy mit bis zu 6 Kohlenstoffatomen substituiert ist,

  oder

- für Formyl oder Nitril steht,

$R^4$ - für Wasserstoff oder
- für einen geradkettigen oder verzweigten Alkylrest mit bis zu vier Kohlenstoffatomen steht, der gegebenenfalls durch ein Sauerstoffatom in der Kette unterbrochen ist und/oder der gegebenenfalls durch einen Piperidino- oder Morpholino-Rest substituiert , ist oder
- für Phenyl oder Benzyl steht,

X - für eine Zahl von 2 bis 15 steht

und

$R^6$ - für einen Rest der Formel

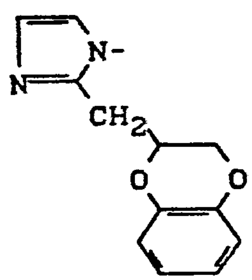

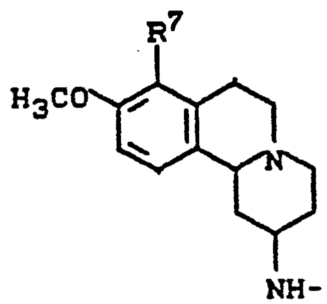

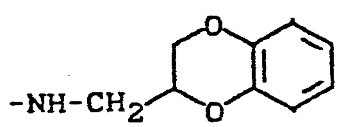

oder

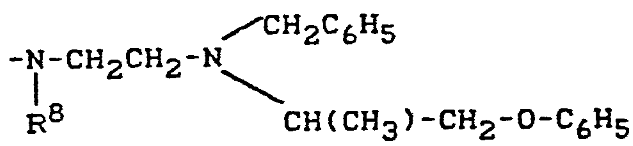

steht,

worin

$R^7$ - für Wasserstoff oder Methoxy steht und

$R^8$ - für Wasserstoff,

- für geradkettiges oder verzweigtes $C_1$-$C_6$-Alkyl oder
- für die Gruppe der Formel

$$-CH_2-CH_2-N\begin{matrix} \diagup CH_2-C_6H_5 \\ \diagdown CH(CH_3)-CH_2-O-C_6H_5 \end{matrix}$$ steht,

und deren physiologisch unbedenklichen Salze.

Bevorzugt sind Verbindungen der Formel (I) in welcher

$R^1$ - für Phenyl, Thienyl, Furyl, Pyridyl, Chinolyl oder Benzoxadiazolyl steht, wobei die genannten Ringsysteme gegebenenfalls durch ein bis zwei gleiche oder verschiedene Substituenten aus der Gruppe Phenyl, $C_1$-$C_2$-Alkyl, $C_1$-$C_2$-Alkoxy, $C_1$-$C_2$-Alkylthio, Dioxymethylen, Fluor, Chlor, Brom oder Iod, Trifluormethyl, Trifluormethoxy, Difluormethoxy, Nitro oder Cyano substituiert sind,

$R^2$ - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoffatom in der Kette unterbrochen ist und/oder der gegebenenfalls substituiert ist durch Fluor, Chlor, Brom, Iod, Cyano, Acetoxy, Hydroxy oder durch eine gegebenenfalls durch Fluor, Chlor, $C_1$-$C_2$-Alkyl, $C_1$-$C_2$-Alkoxy oder Trifluormethyl substituierte Phenyl- oder Phenoxygruppe, oder durch eine α-, β- oder γ-Pyridylgruppe, oder durch eine tertiäre Aminogruppe, wobei diese Aminogruppe zwei gleiche oder verschiedene Substituenten aus der Gruppe $C_1$-$C_4$-Alkyl, Phenyl oder Benzyl trägt,

$R^3$ und $R^5$ gleich oder verschieden sind und jeweils

- für einen geradkettigen, verzweigten oder cyclischen Alkylrest mit bis zu 6 Kohlenstoffatomen stehen,

  oder

  einer der Substituenten $R^3$ oder $R^5$ einen Alkylrest mit bis zu 4 Kohlenstoffatomen darstellt, der durch Acetoxy, Methoxy, Dimethoxy, Hydroxy, Amino, Phthalimido, Aminoalkoxy oder Phthalimidoalkoxy mit jeweils bis zu 4 Kohlenstoffatomen je Alkoxygruppe substituiert ist,

  oder

- für Formyl oder Cyano steht,

$R^4$ - für Wasserstoff oder

- für einen geradkettigen oder verzweigten Alkylrest mit bis zu vier Kohlenstoffatomen steht, der gegebenenfalls durch ein Sauerstoffatom in der Kette unterbrochen ist und/oder der gegebenenfalls durch einen Piperidino- oder Morpholino-Rest substituiert ist oder
- für Benzyl steht,

X - für eine Zahl von 2 bis 10 steht, und

$R^6$ - für einen Rest der Formel

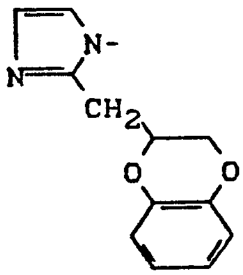

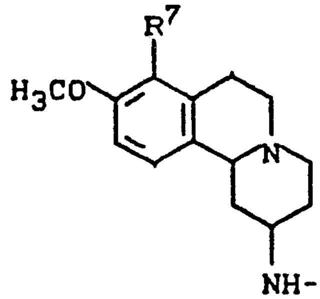

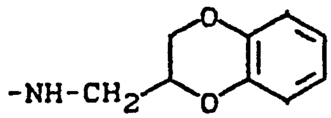

oder

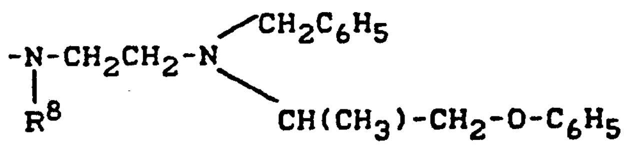

steht,

worin

$R^7$ - für Wasserstoff oder Methoxy steht und

$R^8$ - für Wasserstoff
- für geradkettiges oder verzweigtes $C_1$-$C_4$-Alkyl oder
- für den Rest der Formel

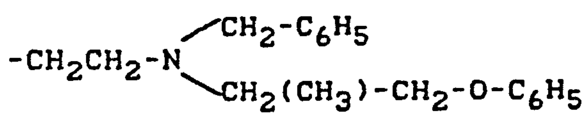

steht,

und ihre physiologisch unbedenklichen Salze.

0236838

Besonders bevorzugt sind solche Verbindungen der allgemeinen Formel (I),

in welcher

$R^1$ - für Phenyl, Pyridyl oder Benzoxadiazolyl steht, wobei der Phenylring durch 1 oder 2 gleiche oder verschiedene Substituenten aus der Gruppe Chlor, Trifluormethyl, Nitro oder Cyano substituiert ist,

$R^2$ - für einen geradkettigen oder verzweigten Kohlenwasserstoffrest mit bis zu 7 Kohlenstoffatomen steht, der gegebenenfalls durch ein Sauerstoffatom in der Kette unterbrochen ist und/oder der gegebenenfalls substituiert ist durch Fluor, Cyano, Phenyl, α-, β- oder γ-Pyridyl oder durch N-Benzyl-N-methylamino,

$R^3$ und $R^5$ für Methyl stehen,

$R^4$ - für Wasserstoff steht,

X - für eine Zahl von 2 bis 6 steht,

und

$R^6$ - für einen Rest der Formel

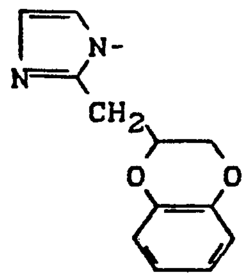

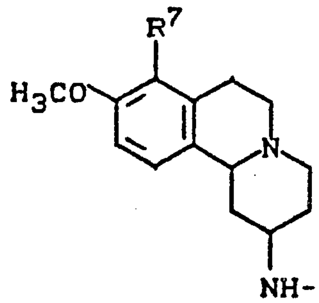

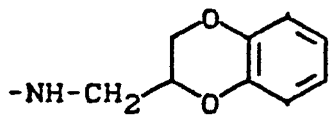

oder

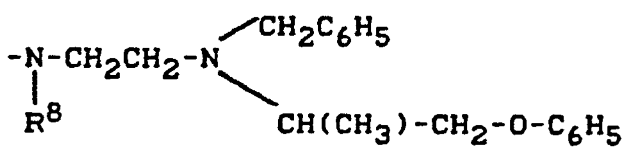

steht,

worin

$R^7$ - für Wasserstoff oder Methoxy steht und

$R^8$ - für Wasserstoff

- für geradkettiges oder verzweigtes $C_1$-$C_4$-Alkyl oder
- für den Rest der Formel

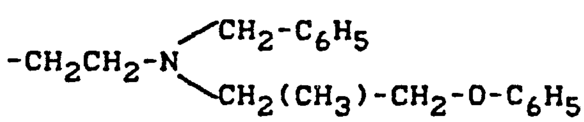

steht,

und ihre physiologisch unbedenklichen Salze.

Physiologisch unbedenkliche Salze sind Salze der erfindungsgemäßen Verbindungen mit anorganischen oder organischen Säuren. Hierzu gehören bevorzugt anorganische Säuren wie Halogenwasserstoffsäuren, bevorzugt HCl oder HBr, Schwefelsäure, Phosphorsäure, oder organische Carbonsäuren oder Sulfonsäuren wie beispielsweise Essigsäure, Maleinsäure, Fumarsäure, Äpfelsäure, Citronensäure, Weinsäure, Milchsäure, Benzoesäure, Methansulfonsäure, Ethansulfonsäure, Benzolsulfonsäure, Naphthalindisulfonsäure oder Toluolsulfonsäure.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diasteromeren in bekannter Weise in die stereoisomer einheitlichen Bestandteile trennen (E. Eliel, Stereochemistry of Carbon Compounds McGraw Hill, 1962).

Die erfindungsgemäßen Aminoester-Dihydropyridine der allgemeinen Formel (I) werden hergestellt, indem man Verbindungen der allgemeinen Formel (II)

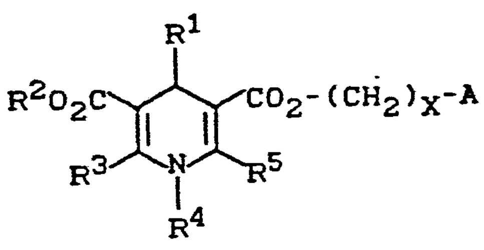

(II)

in welcher

$R^1$-$R^5$ und X die oben angegebene Bedeutung haben und

A für den Rest $NHR^8$ steht, wobei $R^8$ die oben angegebene Bedeutung hat, oder für Halogen steht,

mit Verbindungen der Formel (III)

$$B\text{-}R^6 \qquad (III)$$

in welcher

$R^6$ die oben angegebene Bedeutung hat und

B für einen reaktiven Rest aus der Gruppe Halogen, Carbonyl oder eine sekundäre Aminogruppe steht,

in inerten organischen Lösungsmitteln bei Temperaturen von 0 bis 200°C umsetzt und bei der Verwendung von Ausgangsverbindungen der Formel (III) mit reaktiven Carbonylgruppen die erhaltenen Schiff'schen Basen nach üblichen Methoden reduziert.

Als bevorzugte Verfahrensvarianten für bestimmte Substituenten der erfindungsgemäßen Verbindungen der allgemeinen Formel (I) seien folgende bevorzugte Ausführungsformen des erfindungsgemäßen Verfahrens genannt:

A) Die erfindungsgemäßen Verbindungen der allgemeinen Formel (Ia)

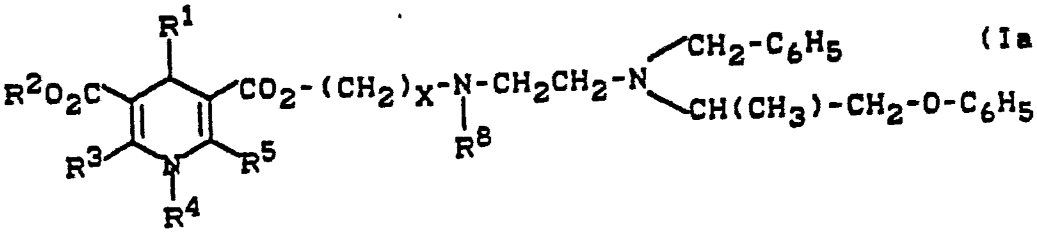

(Ia)

in welcher
$R^1$-$R^5$,$R^8$ und X die angegebene Bedeutung haben,
erhält man,

indem man Amino-Verbindungen der allgemeinen Formel (IIa)

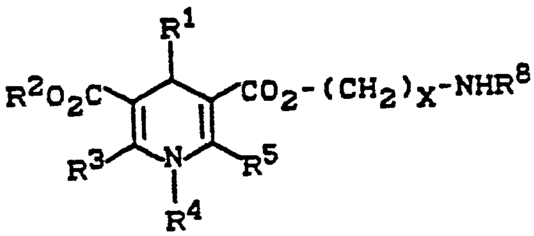

(IIa)

in welcher
$R^1$-$R^5$, X und $R^8$ die angegebene Bedeutung haben,

mit Halogenverbindungen der allgemeinen Formel (IIIa)

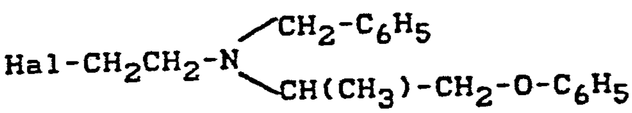

(IIIa)

in welcher

Hal - für Fluor, Chlor, Brom oder Iod, bevorzugt für Chlor oder Brom steht,

in inerten organischen Lösungsmitteln, gegebenenfalls in Anwesenheit einer Base umsetzt, gegebenenfalls anschließend alkyliert, und gegebenenfalls mit Säuren die physiologisch unbedenklichen Salze herstellt.

Je nach Art der verwendeten Ausgangsverbindungen läßt sich das erfindungsgemäße Verfahren durch folgendes Reaktionsschema verdeutlichen:

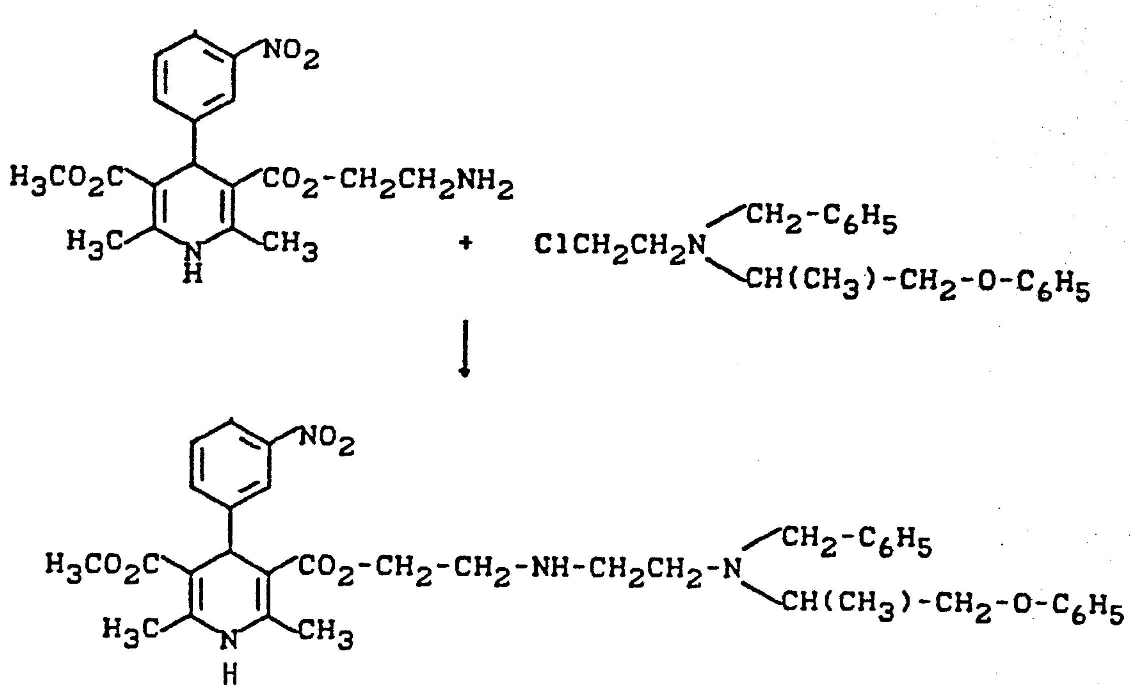

Die als Ausgangsstoffe eingesetzten Aminoverbindungen (IIa) sind bekannt oder können nach bekannten Methoden hergestellt werden [US-PS 3.985.758;EP 151 006].

Die als Ausgangsstoffe verwendeten Halogenverbindungen der Formel (IIIa) sind bekannt oder können nach bekannten Methoden hergestellt werden [Biochem. Pharmacol., 30(12), 1685-92].

Als Lösungsmittel können die üblichen organischen Lösungsmittel verwendet werden, die sich unter den Reaktionsbedingungen nicht verändern. Hierzu gehören bevorzugt Alkohole wie Methanol, Ethanol, Propanol, Isopropanol, Ether wie Diethylether, Dioxan oder Tetrahydrofuran, Dimethylformamid, Dimethylsulfoxid, Acetonitril, Essigester, Hexamethylphosphorsäuretriamid, Pyridin, Picolin, N-Methylpiperidin oder Kohlenwasserstoffe wie Benzol, Toluol, oder Xylol. Ebenso können Gemische der genannten Lösungsmittel eingesetzt werden.

Als Basen eigenen sich die üblichen anorganischen oder organischen Basen. Hierzu gehören bevorzugt Alkalihydroxide wie Natrium- oder Kaliumhydroxid, Alkalicarbonate wie Natrium- oder Kaliumcarbonate, Alkalialkoholate wie Natrium- oder Kaliummethanolat, Natrium- oder Kaliumethanolat, oder organische Amine wie Trialkylamine, z.B. Triethylamin, Pyridin, Picolin, N-Methylpiperidin, oder Amide wie Natriumamid oder Lithiumdiisopropylamid, oder Metallorganyle wie z.B. Butyllithium oder Phenyllithium.

Die Umsetzung wird im allgemeinen in einem Temperaturbereich von 0°C bis 200°C, bevorzugt von Raumtemperatur bis zur Siedetemperatur des jeweiligen Lösungsmittels durchgeführt.

Die Reaktion wird im allgemeinen bei Normaldruck durchgeführt. Es ist jedoch ebenfalls möglich, die Reaktion bei erhöhtem oder erniedrigtem Druck durchzuführen.

Für die Synthese der Verbindungen der Formel (Ia) in welcher $R^8$ für Wasserstoff oder $C_1$-$C_6$-Alkyl steht, werden die entsprechenden Amino-Verbindungen der Formel (IIa) vorzugsweise in molaren bis doppelt molaren Mengen eingesetzt. Für die Synthese der Verbindungen der Formel (Ia) in welcher $R^8$ für die Gruppe der Formel

$$-CH_2CH_2-N\begin{matrix} \diagup CH_2-C_6H_5 \\ \diagdown CH(CH_3)-CH_2-O-C_6H_5 \end{matrix}$$ steht,

wird die entsprechende Halogenverbindung der Formel (IIIa) vorzugsweise in doppelt bis dreifach molaren Mengen eingesetzt.

Die Base wird in einer Menge von 1 bis 100 mol, bevorzugt von 1 bis 50 mol bezogen auf 1 mol Aminoverbindung eingesetzt.

Eine anschließende Alkylierung erfolgt gegebenenfalls nach an sich bekannten Methoden in inerten Lösungsmitteln wie Alkoholen, z.B. Methanol, Ethanol oder n- oder i-Propanol, Ethern z.B. Diethylether, Dioxan oder Tetrahydrofuran, oder Halogenkohlenwaserstoffen wie Methylenchlorid, Chloroform oder Tetrachlorkohlenstoff, oder Kohlenwasserstoffen wie Benzol, Toluol oder Xylol oder Hexan, mit Alkylierungsmitteln wie Alkylhalogeniden, bevorzugt Alkylbromiden oder Alkyliodiden, Dialkylsulfaten oder Diazoalkanen, gegebenenfalls mit Basen wie NaOH, KOH, Natrium-oder Kaliumhydroxid oder organischen Basen wie Triethylamin oder Pyridin, wie es beispielsweise ausführlich in "Organikum", 11. Auflage, VFB Deutscher Verlag der Wissenschaften Berlin 1972, Seite 228 beschrieben wird.

B) Die Verbindungen der allgemeinen Formel (Ib)

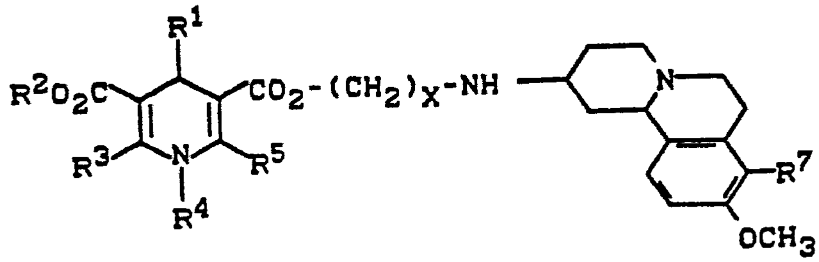

(Ib),

in welcher
$R^1$-$R^5$, $R^7$ und X die angegebene Bedeutung haben, werden hergestellt, indem man

in einem ersten Schritt Ketone der Formel (IIIb)

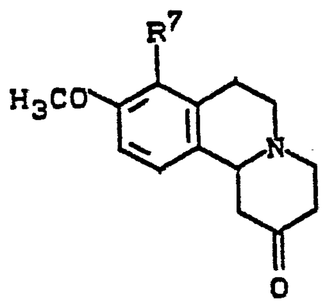

(IIIb)

mit Aminen der Formel (IIa)
in welchen
$R^1$-$R^5$, $R^7$ und X die angegebene Bedeutung haben,
und
$R^8$ - für Wasserstoff steht,

in einem inerten organischen Lösungsmittel gegebenenfalls in Gegenwart eines Katalysators umsetzt, die so erhaltenen Schiffschen Basen in einem zweiten Schritt in inerten Lösungsmitteln reduziert und gegebenenfalls anschließend mit Säuren die pharmakologisch unbedenklichen Salze herstellt.

Verwendet man als Ausgangstoffe 1,4-Dihydro-2,6-dimethyl-4-(3-nitrophenyl)-pyridin-3,5-dicarbonsäure-3-(2-aminoethyl)-5-methylester und 1,3,4,6,7,11b-Hexahydro-8,9-dimethoxy-2-oxo-2H-benzo[a]chinolizin der Formel (IIb), so läßt sich die Synthese der erfindungsgemäßen Verbindungen der Formel (Ib) durch folgendes Schema verdeutlichen:

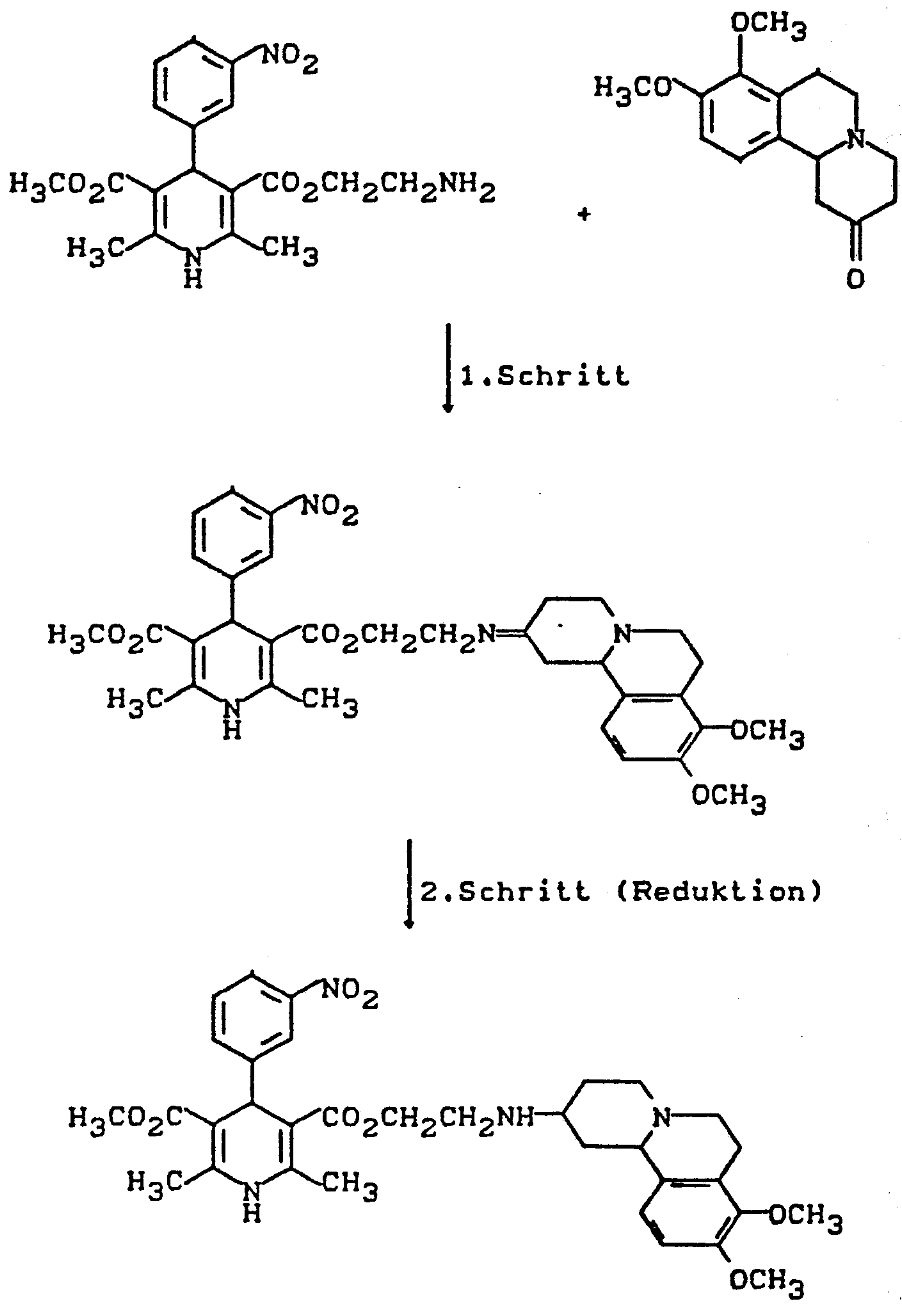

Die als Ausgangsmaterial verwendeten Ketone der Formel (IIIb) sind neu. Sie können nach bekannten Methoden hergestellt werden, wie es beispielsweise von D. Beke und C. Szantay in Chem. Ber. 95, 2132 (1962) beschrieben wird.

Die als Ausgangsstoffe verwendeten Verbindungen der allgemeinen Formel (IIa) sind bekannt oder können nach bekannten Methoden hergestellt werden [US-PS 3.985.758; EP 151.006].

Die Herstellung der Schiffschen Basen im ersten Schritt erfolgt in an sich bekannter Weise in inerten organischen Lösungsmitteln, gegebenenfalls in Anwesenheit eines Katalysators und gegebenenfalls in Anwesenheit eines wasserbindenden Mittels. Das erfindungsgemäße Verfahren kann in zwei Schritten d.h. mit Isolierung der Schiffschen Basen, durchgeführt werden. Ebenso ist es möglich, die Reaktion als Eintopfverfahren durchzuführen.

Als Verdünnungsmittel eignen sich hierbei die üblichen organischen Lösungsmittel, die unter den Reaktionsbedingungen nicht verändert werden. Hierzu gehören bevorzugt Alkohole wie Methanol, Ethanol, Propanol oder Isopropanol, Ether wie Diethylether, Dioxan oder Tetrahydrofuran, Halogenkohlenwasserstoffe wie beispielsweise Methylenchlorid, Chloroform oder Tetrachlorkohlenstoff, Dimethylformamid, oder aromatische Kohlenwasserstoffe wie beispielsweise Benzol, Toluol oder Xylol. Außerdem ist es möglich, Gemische der genannten Lösungsmittel einzusetzen.

Als Katalysatoren werden im allgemeinen Säuren verwendet. Hierzu gehören bevorzugt anorganische Säuren wie z.B. Salzsäure oder Schwefelsäure, oder organische Säuren, wie z.B. Methan-, Ethan-, Benzol- oder Toluolsulfonsäure oder Essigsäure. Ebenso ist es möglich Essigsäure im Gemisch mit Acetanhydrid als wasserentziehendes Mittel einzusetzen. Das bei der Reaktion gebildete Wasser kann gegebenenfalls im Gemisch mit dem verwendeten Lösungsmittel während oder nach der Reaktion, z.B. durch Destillation, oder durch Zugabe von wasserbindenden Mitteln wie z.B. Phosphorpentoxid oder bevorzugt durch Molekularsieb entfernt werden.

Die Durchführung der Reaktion erfolgt im allgemeinen bei einer Temperatur im Bereich von 0°C bis 150°C, bevorzugt von 20°C bis zur Siedetemperatur des jeweiligen Lösungsmittels.

Die Umsetzung kann bei normalem, erhöhtem oder erniedrigtem Druck durchgeführt werden. Im allgemeinen arbeitet man bei Normaldruck.

Bei der Durchführung der Reaktion werden im allgemeinen die Ausgangsstoffe in einem molaren Verhältnis von Keton IIIb zu Amin (IIa) von 0,5 : 2 bis 1 : 2 eingesetzt. Bevorzugt verwendet man molare Mengen der Reaktanden.

Die Durchführung der Herstellung der Schiffschen Basen aus Keton IIIb und Amin (IIa) ist nicht auf die beschriebenen Varianten beschränkt sondern kann ebenso nach anderen bekannten Methoden durchgeführt werden, die beispielsweise in Houben-Weyl "Methoden der organischen Chemie", Bd. 11/2 beschrieben werden.

Die Reduktion der Schiffschen Basen zu den erfindungsgemäßen Verbindungen der Formel (Ib) erfolgt nach üblichen Methoden entweder durch Wasserstoff in inerten organischen Lösungsmitteln wie Alkoholen, Ethern oder Halogenkohlenwasserstoffen mit Katalysatoren wie Raney-Nickel, Palladium, Palladium auf Tierkohle oder Platin, oder mit Hydriden in inerten organischen Lösungsmitteln, gegebenenfalls in Anwesenheit eines Katalysators.

Bevorzugt wird die Reaktion mit Hydriden wie komplexe Alkaliborhydriden oder Aluminiumhydriden durchgeführt. Bevorzugt werden hierbei Natriumborhydrid, Lithiumaluminiumhydrid oder Cyanoborhydrid eingesetzt.

Als Lösungsmittel eigenen sich hierzu alle inerten organischen Lösungsmittel, die sich unter den Reaktionsbedingungen nicht verändern. Hierzu gehören bevorzugt Alkohole wie Methanol, Ethanol, Propanol oder Isopropanol, Ether wie Diethylether, Dioxan oder Tetrahydrofuran oder Dimethylformamid.

Als Katalysatoren werden im allgemeinen Säuren verwendet. Hierzu gehören bevorzugt anorganische Säuren wie Salzsäure oder Schwefelsäure, oder organische Säuren wie Carbonsäuren oder Sulfonsäuren, z.B. Essigsäure, Trifluoressigsäure, Methan-, Ethan-, Benzol- oder Toluolsulfonsäure.

C) Die erfindungsgemäßen Verbindungen der allgemeinen Formel (Ic)

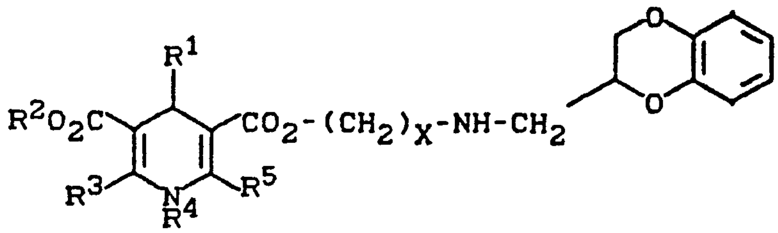

(Ic)

in welcher
$R^1$-$R^5$ und X die angegebene Bedeutung haben

erhält man, wenn man

[1] Amino-Dihydropyridine der allgemeinen Formel (IIa)

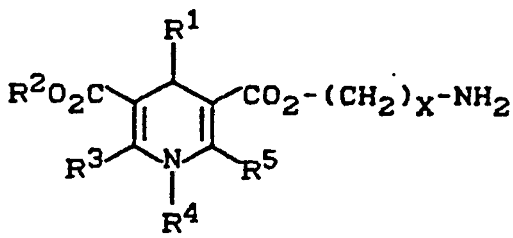

(IIa)

in welcher
$R^1$-$R^5$ und X die angegebene Bedeutung haben,

mit Halogenverbindungen der Formel (IIIc)

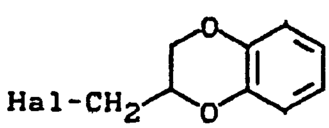

(IIIc)

0236838

in welcher

Hal- für Fluor, Chlor, Brom oder Iod, bevorzugt für Chlor oder Brom steht,

in inerten organischen Lösungsmitteln, gegebennfalls in Anwesenheit einer Base umsetzt, und gegebenenfalls mit Säuren die physiologisch unbedenklichen Salze herstellt,

oder wenn man

[2] Amino-Dihydropyridine der Formel (IIa) mit Carbonylverbindungen der Formel (IIIcc)

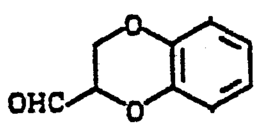

(IIIcc)

in einem inerten Lösungsmittel gegebenenfalls in Gegenwart eines Katalysators umsetzt, die so erhaltenen Schiffschen Basen in einem zweiten Schritt in inerten Lösungsmitteln reduziert und gegebenenfalls anschließend mit Säuren die physiologisch unbedenklichen Salze herstellt.

Je nach Art der verwendeten Ausgangsstoffe lassen sich Verfahren C1 und C2 durch folgendes Schema verdeutlichen:

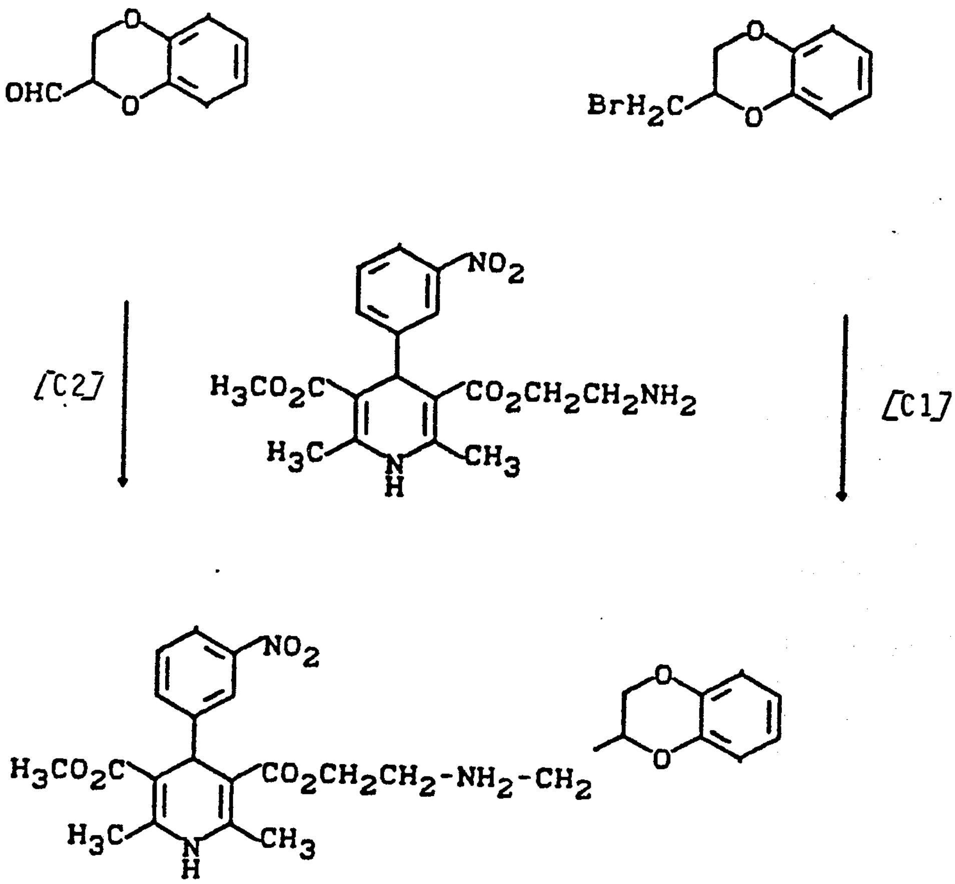

Die als Ausgangsstoffe eingesetzten Amino-Dihydropyridine (IIa) sind bekannt oder können nach bekannten Methoden hergestellt werden [US-PS 3.985.758; EP 151 006].

Die als Ausgangsstoffe eingesetzten Halogenverbindungen (IIIc) sind bekannt oder können nach bekannten Methoden hergestellt werden [DE-OS 3 124 366].

Die als Ausgangsstoff eingesetzte Carbonylverbindung der Formel (IIIcc) ist bekannt [Petragnani et al., Il Farmaco -Ed.Sc.-vol.32-fasc. 7,512].

Als Lösungsmittel für Verfahren C1 eignen sich die üblichen organischen Lösungsmittel, die sich unter den Reaktionsbedingungen nicht verändern. Hierzu gehören bevorzugt Alkohole wie Methanol, Ethanol, Propanol, Isopropanol, Ether wie Diethylether, Dioxan oder Tetrahydrofuran, Dimethylformamid, Dimethylsulfoxid, Acetonitril, Essigester, Hexamethylphosphorsäuretriamid, Pyridin, Picolin, N-Methylpiperidin oder Kohlenwasserstoffe wie Benzol, Toluol oder Xylol. Es ist ebenso möglich, Gemische der genannten Lösungsmittel einzusetzen.

Als Basen für Verfahren C1 eigenen sich die üblichen anorganischen oder organischen Basen. Hierzu gehören bevorzgut Alkalihydroxide wie Natrium- oder Kaliumhydroxid, Alkalicarbonate wie Natrium- oder Kaliumcarbonat, Alkalialkoholate wie Natrium- oder Kaliummethanolat, Natrium- oder Kaliumethanolat, Kalium-tert.-butanolat oder organische Amine wie Trialkylamine,

z.B. Triethylamin, Pyridin, Picolin, N-Methylpiperidin, oder Amide wie Natriumamid oder Lithiumdiisopropylamid, oder Metallorganyle wie z.B. Butyllithium oder Phenyllithium.

Die Umsetzung wird im allgemeinen in einem Temperaturbereich von 0° C bis 200° C, bevorzugt von Raumtemperatur bis zur Siedetemperatur des jeweiligen Lösungsmittels durchgeführt.

Die Reaktion wird im allgemeinen bei Normaldruck durchgeführt. Es ist jedoch ebenfalls möglich, die Reaktion bei erhöhtem oder erniedrigtem Druck durchzuführen.

Bei der Umsetzung setzt man im allgemeinen 0,5 bis 5 mol, bevorzugt 1 bis 2 mol Halogenverbindung (IIIc), und 1 bis 100, bevorzugt 1 bis 50 mol Base bezogen auf 1 Mol Aminoverbindung (IIa) ein.

Die Herstellung der Schiffschen Basen im ersten Schritt des Verfahrens C2 erfolgt in an sich bekannter Weise in inerten organischen Lösungsmitteln, gegebenenfalls in Anwesenheit eines Katalysators und gegebenenfalls in Anwesenheit eines wasserbindenden Mittels. Das erfindungsgemäße Verfahren kann in zwei Schritten d.h. mit Isolierung der Schiffschen Basen, durchgeführt werden. Ebenso ist es möglich, die Reduktion als Eintopfverfahren durchzuführen.

Als Verdünnungsmittel eignen sich hierbei die üblichen organischen Lösungsmittel, die unter den Reaktionsbedingungen nicht verändert werden. Hierzu gehören bevorzugt Alkohole wie Methanol, Ethanol, Propanol oder Isopropanol, Ether wie Diethylether, Dioxan oder Tetrahydrofuran, Halogenkohlenwasserstoffe wie beispielsweise Methylenchlorid, Chloroform oder Tetrachlorkohlenstoff, Dimethylformamid, oder aromatische Kohlenwasserstoffe wie beispielsweise Benzol, Toluol oder Xylol. Außerdem ist es möglich Gemische der genannten Lösungsmittel einzusetzen.

Als Katalysatoren werden im allgemeinen Säuren verwendet. Hierzu gehören bevorzugt anorganische Säuren wie z.B. Salzsäure oder Schwefelsäure, oder organische Säuren, wie z.B. Methan-, Ethan-, Benzol- oder Toluolsulfonsäure oder Essigsäure. Ebenso ist es möglich Essigsäure im Gemisch mit Acetanhydrid als wasserentziehendem Mittel einzusetzen. Das bei der Reaktion gebildete Wasser kann gegebenenfalls im Gemisch mit dem verwendeten Lösungsmittel während oder nach der Reaktion, z.B. durch Destillation, oder durch Zugabe von wasserbindenden Mitteln wie z.B. Phosphorpentoxid oder bevorzugt Molekularsieb entfernt werden.

Die Durchführung der Reaktion erfolgt im allgemeinen bei einer Temperatur im Bereich von 0° C bis 150° C, bevorzugt von 20° C bis zur Siedetemperatur des jeweiligen Lösungsmittels.

Die Umsetzung kann bei normalem, erhöhtem oder erniedrigtem Druck durchgeführt werden. Im allgemeinen arbeitet man bei Normaldruck.

Bei der Durchführung der Reaktion werden im allgemeinen die Ausgangsstoffe in einem molaren Verhältnis von Carbonylverbindung (IIIcc) zu Amin (IIa) von 0,5 : 2 bis 1 : 2 eingesetzt. Bevorzugt verwendet man molare Mengen der Reaktanden.

Die Durchführung der Herstellung der Schiffschen Basen aus der Carbonylverbindung (IIIcc) und Aminen (IIa) ist nicht auf die beschriebene Varianten beschränkt sondern kann ebenso nach anderen bekannten Methoden durchgeführt werden, die beispielsweise in Houben-Weyl, "Methoden der organischen Chemie", Bd. 11/2 beschrieben werden.

Die Reduktion der Schiffschen Basen zu den erfindungsgemäßen Verbindungen der Formel (Ic) erfolgt nach üblichen Methoden entweder durch Wasserstoff in inerten organischen Lösungsmitteln wie Alkoholen, Ethern oder Halogenkohlenwasserstoffen mit Katalysatoren wie Raney-Nickel, Palladium, Palladium auf Tierkohle oder Platin, oder mit Hydriden in inerten organischen Lösungsmitteln, gegebenenfalls in Anwesenheit eines Katalysators.

0236838

Bevorzugt wird die Reduktion mit Hydriden wie komplexe Alkaliborhydriden oder Aluminiumhydriden durchgeführt. Bevorzugt werden hierbei Natriumborhydrid, Lithiumaluminiumhydrid oder Cyanoborhydrid eingesetzt.

Als Lösungsmittel eigenen sich hierzu alle inerten organischen Lösungsmittel, die sich unter den Reaktionsbedingungen nicht verändern. Hierzu gehören bevorzugt Alkohole wie Methanol, Ethanol, Propanol oder Isopropanol, Ether wie Diethylether, Dioxan oder Tetrahydrofuran oder Dimethylformamid.

Als Katalysatoren werden im allgemeinen Säuren verwendet. Hierzu gehören bevorzugt anorganische Säuren wie Salzsäure oder Schwefelsäure, oder organische Säuren wie Carbonsäuren oder Sulfonsäuren, z.B. Essigsäure, Trifluoressigsäure, Methan-, Ethan-, Benzol- oder Toluolsulfonsäure.

D) Die erfindungsgemäßen Verbindungen der allgemeinen Formel (Id)

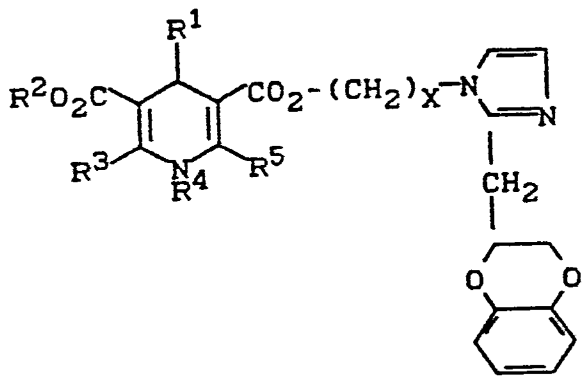

(Id)

in welcher

$R^1$-$R^5$ und X die angegebene Bedeutung haben,

erhält man, wenn man Dihydropyridinhalogenester der allgemeinen Formel (IId)

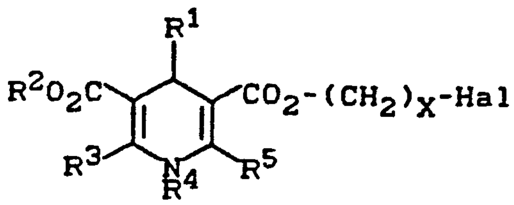

(IId)

in welcher
$R^1$-$R^5$ und X die angegebene Bedeutung haben und

Hal - für Fluor, Chlor, Brom, Iod, bevorzugt für Chlor oder Brom steht,

mit dem Imidazol der Formel (IIId)

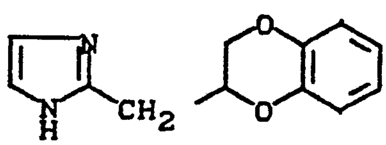

(IIId)

in inerten organischen Lösungsmitteln, gegebenfalls in Anwesenheit einer Base umsetzt.

Die Herstellung der erfindungsgemäßen Verbindungen der Formel (Id) läßt sich durch folgendes Formelschema verdeutlichen:

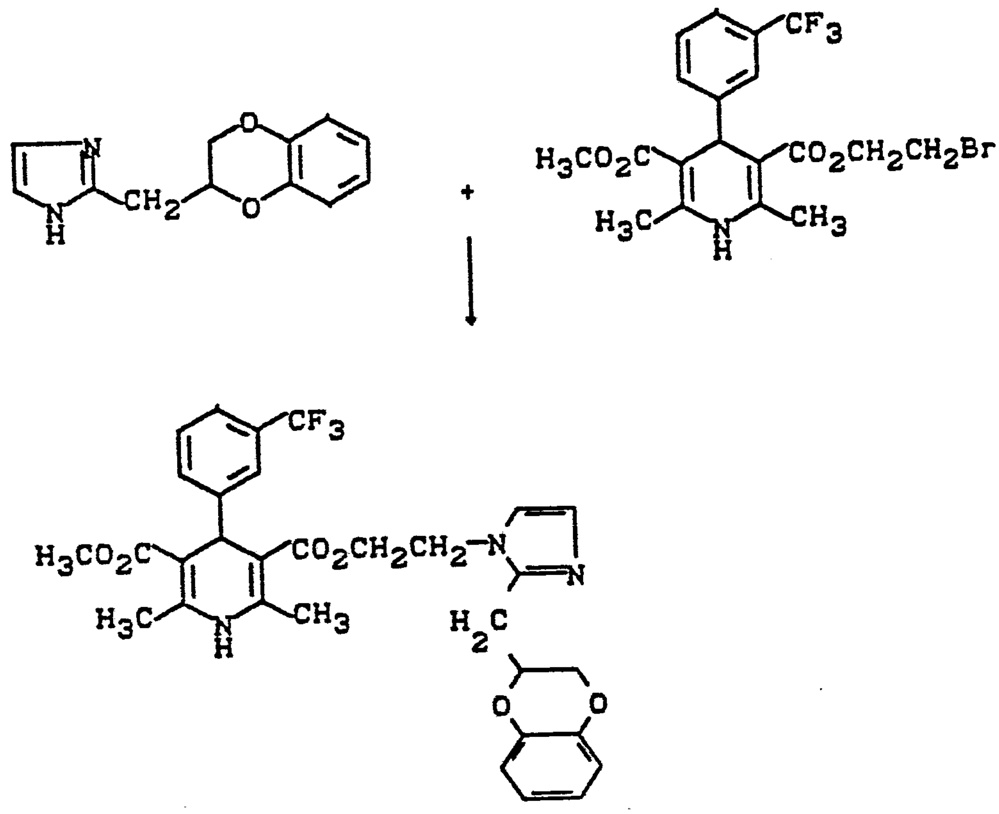

Die als Ausgangsstoffe eingesetzten Dihydropyridinhalogenester (IId) sind bekannt oder können nach bekannten Methoden hergestellt werden [Kuthan et al., Ind. Eng. Chem. Prod. Res. Der. 21, 191 (1982)].

Das als Ausgangsstoff eingesetzte Imidazol (IIId) ist bekannt oder kann nach bekannten Methoden hergestellt werden [US-PS 4 302 469].

Als Lösungsmittel eignen sich die üblichen organischen Lösungsmittel, die sich unter den Reaktionsbedingungen nicht verändern. Hierzu gehören vorzugsweise Ether wie Diethylether, Dioxan, Tetrahydrofuran, Glykolmono- oder -dimethylether, Amide wie Dimethylformamid oder Hexamethylphosphorsäuretriamid, Halogenkohlenwasserstoffe wie Methylenchlorid, Chloroform oder Tetrachlorkohlenstoff, Kohlenwasserstoffe wie Benzol, Toluol oder Xylol, Alkohole wie Methanol, Ethanol, Propanol oder Isopropanol, oder Basen wie Pyridin, Picolin oder N-Methylpiperidin. Ebenso können Gemische der genannten Lösungsmittel eingesetzt werden.

Als Basen eignen sich die üblichen anorganischen oder organischen Basen wie Natrium oder Kalium, Alkalihydride wie Natriumhydrid oder Kaliumhydrid, Alkaliamide wie Natriumamid oder Lithiumdiisopropylamid, lithiumorganische Verbindungen wie Butyllithium oder Phenyllithium, Alkalialkoholate wie Kaliummethanolat oder -ethanolat, Natriummethanolat oder -ethanolat, Kalium-tert.-butanolat, Alkalihydroxide wie Natrium- oder Kaliumhydroxid, oder Alkalicarbonate wie Natrium- oder Kaliumcarbonat, oder organische Amine wie Trialkylamine z.B. Triethylamin, Pyridin, 1,5-Diazabicyclo(4,3,0)non-5-en, 1,5-Diazabicyclo(5,4,0)undec-5-en.

Die Umsetzung erfolgt im allgemeinen in einem Temperaturbereich von -20° bis 100°C, bevorzugt von 0°C bis 80°C. Die Reaktion kann bei Normaldruck, aber auch bei erhöhtem oder erniedrigtem Druck durchgeführt werden. Im allgemeinen arbeitet man bei normalem Druck.

Bei der Durchführung der Reaktion setzt man im allgemeinen 0,5 bis 5 mol, bevorzugt 1 bis 1,5 mol Dihydropyridinhalogenester (IId) sowie 1 bis 50, bevorzugt 1 bis 10 mol Base bezogen auf 1 mol Imidazol (IIId) ein.

Die erfindungsgemäßen Verbindungen eigenen sich als Therapeutika zur Behandlung ischämischer Herzerkrankungen und essentiellem Hochdruck.

Bei der Therapie der ischämischen Herzerkrankungen (verschiedene klinische Formen von Angina pectoris, Herzinfarkt, Herzversagen) beeinflussen sie die zugrunde liegenden pathogenetischen Mechanismen, hauptsächlich die eingeschränkte Verfügbarkeit von Sauerstoff positiv.

Die erfindungsgemäßen Verbindungen zeigen zusätzlich zu der kalziumantagonistischen Wirksamkeit auch einen antagonistisierenden Effekt auf die vaskulären alpha-Adrenorezeptoren (alpha-Blockade). Demzufolge wird das therapeutische Wirkspektrum dieser Stoffe um das venöse pooling bzw. eine herzmuskelschonende Herabsetzung der Vorlast ergänzt. Dies verbessert die therapeutische Wirksamkeit und ermöglicht es, eine größere Zahl von Patienten therapeutisch zu erfassen.

Die gleiche positive Wirkung zeigen die erfindungsgemäßen Verbindungen auch bei der Behandlung der Hochdruckkrankheit. Durch die zusätzliche Senkung der Vorlast des Herzens ("preload") werden mit Erfolg auch Hochdruckkranke mit hyperdynamischer Kreislaufregulation therapiert, die mit den herkömmlichen Präparaten nur zum Teil erfaßt werden können. Die venendilatierende, herzentlastende Therapie wird bei Hochdruckkranken auch zur Minderung der Herzhypertrophie, Verbesserung der Herzleistung bei Herzinsuffizienz und zur Senkung des Sauerstoffbedarf schwerkranker Hypertoniker führen. Somit werden die therapeutischen Möglichkeiten für diese chronischen, weitverbreiteten Herzkreislauferkrankungen erheblich verbessert.

Die spezifische kalziumantagonistische Wirkung der erfindungsgemäßen Verbindungen wird in einem Test an isolierten Kaninchengefäßen (Aorta) geprüft. In diesem Test werden Kontraktionen des Gefäßmuskels in vitro durch Stimulation mit KCl oder durch Noradrenalin hervorgerufen. KCl verursacht Kontraktionen durch den depolariationsabhängigen Einwärtsstrom von Kalziumionen.

Diese Kontraktionen werden durch kalziumantagonistische Pharmaka dosenabhängig gehemmt. Noradrenalin verursacht Kontraktionen durch Erregung der vaskulären alpha-Rezeptoren und dadurch bedingte Freisetzung der Kalziumionen aus den intrazellulären Speichern. Die Noradrenalin-induzierten Kontraktionen werden durch spezifische Kalziumantagonisten nicht gehemmt. Antagonisten der alpha-Adrenorezeptoren dagegen hemmen nur die Noradrenalin-induzierte Kontraktion.

Die erfindungsgemäßen Verbindungen hemmen überraschenderweise sowohl die Depolarisation-(KCl)-induzierten als auch die Noradrenalin-induzierten Kontraktionen des isolierten Gefäßmuskels von Kaninchen (s. Tabelle 1).

An narkotisierten Katzen (Ketamin) wird das Venenvolumen einer Extremität mittels einer Plethysmographie gemessen. Gleichzeitig wird auch der arterielle Blutdruck registriert. Bisher bekannte kalziumantagonistische DHP-Derivate senken den arteriellen Blutdruck und vermindern das Venenvolumen durch reflektorische Venenkonstriktion. Antagonisten der adrenergen alpha-Rezeptoren wie Phentolamin, Prazosin oder Yohimbin führen zur Erhöhung des Venenvolumens durch Senkung des adrenergen Venentonus.

Die erfindungsgemäßen Verbindungen senken den arteriellen Blutdruck mit gleichzeitiger Steigerung des Venenvolumens und verursachen dadurch das venöse pooling im Zusatz zu der arteriellen Gefäßerweiterung (siehe Tabelle 2).

Die erfindungsgemäßen Verbindungen sind auch nach oraler Applikation wirksam. An wachen Ratten mit genetisch bedingtem Hochdruck ("spontan hypertone Ratten" des Okamoto-Stammes) wird der arterielle Blutdruck mit der "Schwanz-Manschette" in definierten Zeitabständen nach Substanzgabe unblutig gemessen. Die zu prüfenden Substanzen werden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung (siehe Tabelle 3).

0236838

Tabelle 1: Wirkstoff-Konzentration bei welcher die Kontraktion des Aortenrings um 50 % gehemmt wird ($IC_{50}$)
Isolierter Aortenring von Kaninchen, stimuliert
a) durch KCl-Depolarisierung oder
b) durch Noradrenalin

| Bsp.-Nr. | $IC_{50}$ a | $IC_{50}$ b |
|---|---|---|
| 1 | 0,23 mg/l | >10 mg/l |
| 2 | 0,9 mg/l | 3,0 mg/l |
| 3 | 0,23 mg/l | >10 mg/l |
| 4 | 0.4 mg/l | 5,0 mg/l |
| 6 | 0,26 mg/l | 0,36 mg/l |
| 7 | 0,5 mg/l | 1,4 mg/l |
| 8 | 0,3 mg/l | 1,6 mg/l |
| 11 | 0,23 mg/l | 0,18 mg/l |
| 12 | 0,016 mg/l | 0,4 mg/l |
| 13 | 0,08 mg/l | 0,7 mg/l |

Tabelle 2: Signifikante Erhöhung der Venenkapazität bei der anästhesierten Katze im Vergleich zum Referenz-Wirkstoff Natriumnitroprussid.

| Bsp.-Nr. | Dosis [mg/kg i.v.] |
|---|---|
| 9 | 1,0 |
| 11 | 0,1 |
| 12 | 0,1 |

Tabelle 3: Blutdrucksenkung an der hypertonen Ratte um 20 mm Hg. ($ED_{20}$)

| Bsp.-Nr. | $ED_{20}$ [mg/kg per os] |
|---|---|
| 6 | 1,0 |
| 7 | 31,5 |
| 8 | 31,5 |
| 11 | 3,0 |
| 13 | 3,0 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkyl-Sulfonate, Aryl-Sulfonate), Dispergiermittel (z. B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Falls nicht ausdrücklich anders angegeben, wurden die $R_f$-Werte der folgenden Beispiele unter Verwendung von DC-Fertigplatten, Kieselgel 60 $F_{254}$ der Firma E. Merck, Darmstadt ermittelt.

Beispiel 1

1,4-Dihydro-2,6-dimethyl-4-(3-nitrophenyl)-pyridin-3,5-dicarbonsäure-3-methylester-5-{6-[2-[N-benzyl-N-(2-phenoxy-1-methylethyl)amino]ethylamino]hexyl}ester

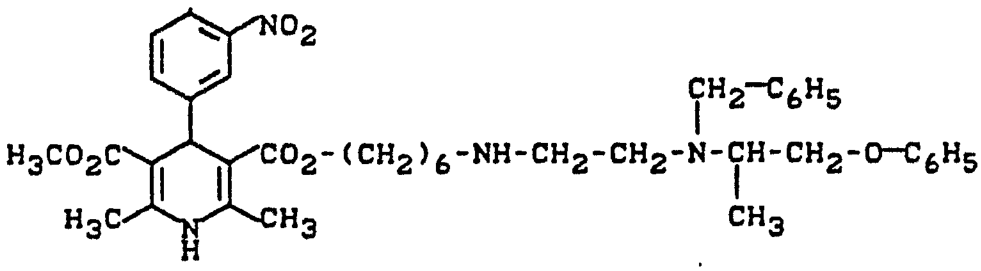

1,5 g (0,0035 Mol) 1,4-Dihydro-2,6-dimethyl-4-(3-nitrophenyl)pyridin-3,5-dicarbonsäure-3-methyl-5-(6-aminohexyl)ester werden mit 1,1 g (0,0035 Mol) N-Benzyl-N-phenoxyisopropyl-β-chlorethylamin und 1,2 g Kaliumcarbonat in 60 ml Dioxan p.a. 20 h am Rückfluß gekocht. Das Reaktionsgemisch wird abgekühlt, am Rotationsverdampfer eingeengt und der Rückstand an einer Kieselgelsäule (Chloroform:Methanol / 10:1) gereinigt. Das erkaltete Öl wird im Hochvakuum getrocknet. Durch Fraktionierung erhält man 540 mg Ausbeute (23% der Theorie) als amorphes Pulver.
$R_f$: 0,18 (10:1/$CHCl_3$:$CH_3OH$)

Beispiel 2

1,4-Dihydro-2,6-dimethyl-4-(3-nitrophenyl)-pyridin-3,5-dicarbonsäure-3-methylester-5-{6-[bis-[2-[N-benzyl-N-(2-phenoxy-1-methylethyl)amino]ethyl]amino]hexyl}ester

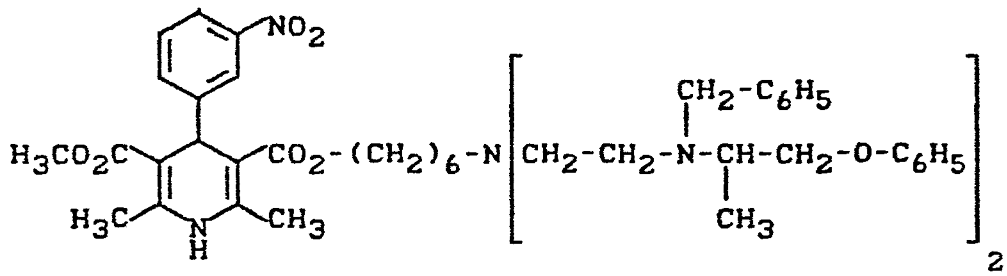

Aus dem Reaktionsgemisch, das bei der Herstellung des Beispiels 1 anfiel, werden bei der Fraktionierung 490 mg der Verbindung des Beispiels 2 in Form eines amorphen Pulvers erhalten.

$R_f$: 0,42 (10:1/$CHCl_3$:$CH_3OH$)

Beispiel 3

1,4-Dihydro-2,6-dimethyl-4-(3-nitrophenyl)pyridin-3,5-dicarbonsäure-3-isopropylester-5-{2-[2-[N-benzyl-N-(2-phenoxy-1-methylethyl)amino]ethylamino]ethyl}ester

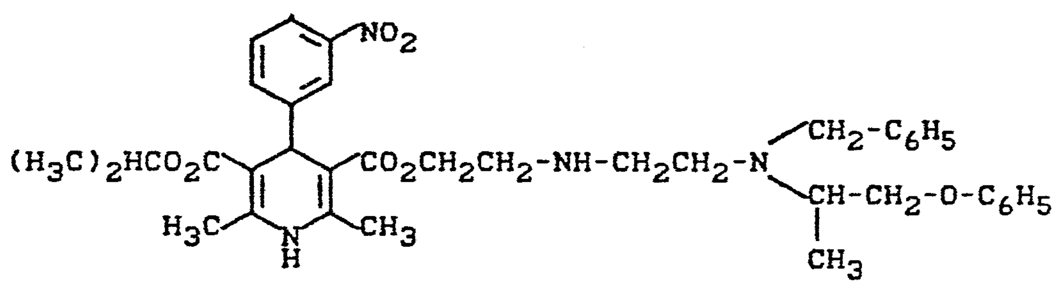

Ausbeute: 27% der Theorie

Schmelzpunkt: amorph.

$R_f$: 0,42 (10:1/$CHCl_3$:$CH_3OH$)

Beispiel 4

1,4-Dihydro-2,6-dimethyl-4-(3-nitrophenyl)-pyridin-3,5-dicarbonsäure-3-isopropylester-5-{2-[bis-[2-[N-benzyl-N-(2-phenoxy-1-methylethyl)amino]ethyl]amino]ethyl}ester

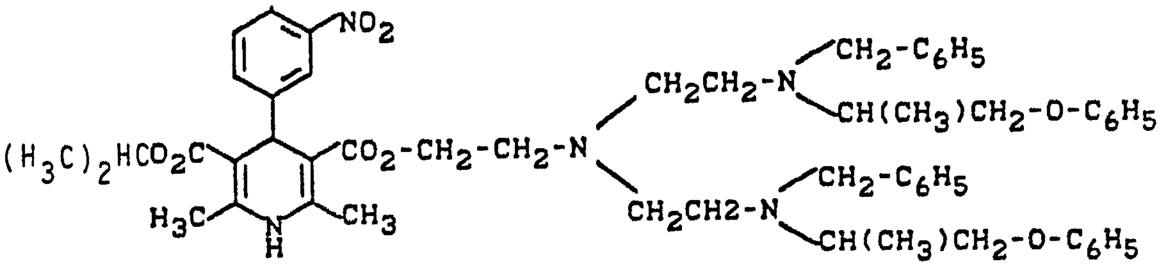

Ausbeute: 26% der Theorie

Schmelzpunkt: amorph.

$R_f$: 0,8 (10:1/$CHCl_3$:$CH_3OH$)

Beispiel 5

1,4-Dihydro-2,6-dimethyl-4-(3-nitrophenyl)pyridin-3,5-dicarbonsäure-3-methylester-5-[6-(1,3,4,6,7,11b-hexahydro-8,9-dimethoxy-2H-benzo[a]chinolizin-2-yl-amino)hexyl]ester

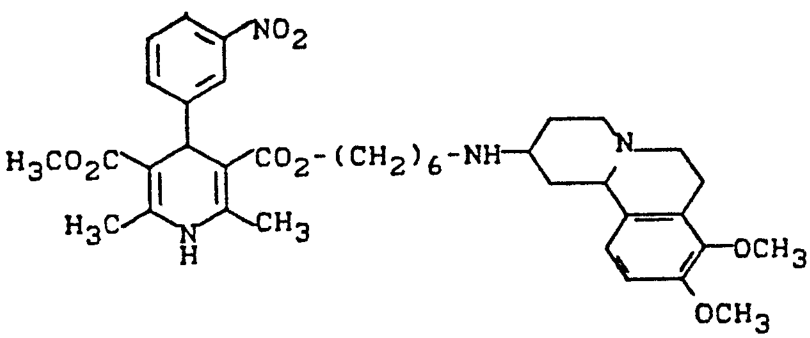

1,3 g (0,005 Mol) 1,3,4,6,7,11b-Hexahydro-8,9-dimethoxy-benzo[a]chinolizin-2-on werden in 30 ml Methanol gelöst und mit methanolischer Salzsäure auf pH 6 eingestellt. Zu dieser Lösung gibt man 2,1 g (0,005 Mol) 1,4-Dihydro-2,6-dimethyl-4-(3-nitrophenyl)pyridin-3,5-dicarbonsäure-3-methylester-5-(6-aminohexyl)ester in Methanol und rührt 2,5 h bei Raumtemperatur mit Molekularsieb 3 Å. Anschließend wird portionsweise 320 mg Natriumcyanoborhydrid zugegeben und 20 h bei Raumtemperatur gerührt. Das Reaktionsgemisch wird filtriert, das Filtrat angesäuert und am Rotationsverdampfer eingeengt. Der Rückstand wird mit Dichlormethan versetzt, mit verdünnter Natronlauge alkalisch gestellt, die organische Phase mit Wasser gewaschen, über Natriumsulfat getrocknet, filtriert und im Vakuum eingeengt. Der Rückstand wird an einer Kieselgelsäule (Chloroform : Methanol :Triethylamin / 10:0,5:0,25) gereinigt. Das so erhaltene Produkt wird anschließend im Hochvakuum getrocknet.

Ausbeute: 1,6 g (48% der Theorie) amorph.

$R_f$: 0,76 (1:1/$CHCl_3$:$CH_3OH$)

Analog Beispiel 5 werden hergestellt:

Beispiel 6

1,4-Dihydro-2,6-dimethyl-4-(3-nitrophenyl)pyridin-3,5-dicarbonsäure-3-methylester-5-[2-(1,3,4,6,7,11b-hexahydro-8,9-dimethoxy-2H-benzo[a]chinolizin-2-yl-amino)ethyl]ester

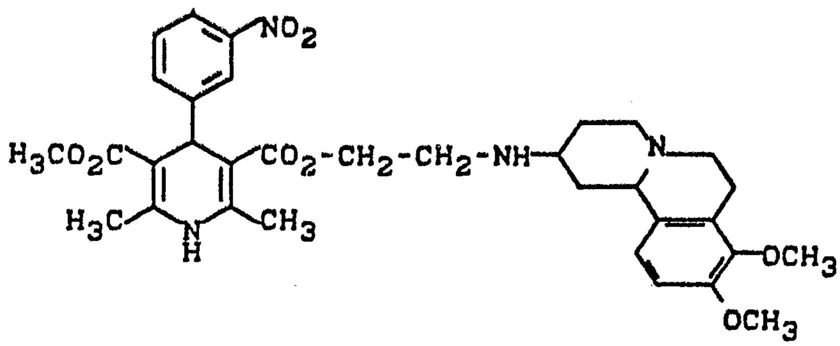

Ausbeute: 48% der Theorie
Schmelzpunkt: amorph.
$R_f$: 0,3 (20:1:0,5/$CHCl_3$:$CH_3OH$:$(C_2H_5)_3N$)

Beispiel 7

1,4-Dihydro-2,6-dimethyl-4-(3-nitrophenyl)pyridin-3,5-dicarbonsäure-3-isopropylester-5-[3-(1,3,4,6,7,11b-hexahydro-8,9-dimethoxy-2H-benzo[a]chinolizin-2-yl-amino)propyl]ester

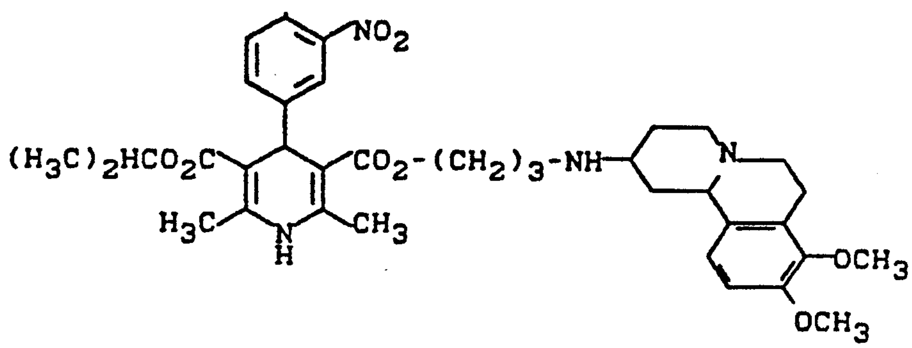

Ausbeute: 47% der Theorie
Schmelzpunkt: amorph.
$R_f$: 0,76(1:1/$CHCl_3$:$CH_3OH$)

Beispiel 8

1,4-Dihydro-2,6-dimethyl-4-(3-nitrophenyl)pyridin-3,5-dicarbonsäure-3-isopropylester-5-[2-(1,3,4,6,7,11b-hexahydro-8,9-dimethoxy-2H-benzo[a]chinolizin-2-yl-amino)ethyl]ester

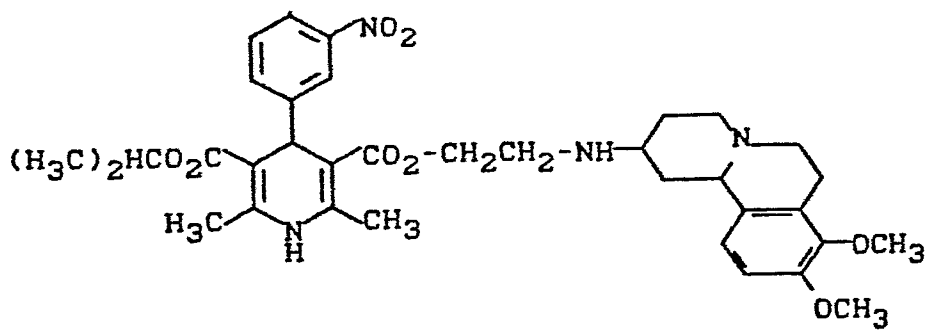

Ausbeute: 47% der Theorie
Schmelzpunkt: amorph.
$R_f$: 0,33(20:1:0,5/$CHCl_3$:$CH_3OH$:$(C_2H_5)_3N$)

Beispiel 9

1,4-Dihydro-2,6-dimethyl-4-(3-nitrophenyl)pyridin-3,5-dicarbonsäure-3-isopropylester-5-[2-(1,3,4,6,7,11b-hexahydro-9-methoxy-2H-benzo[a]chinolizin-2-yl)amino]ethylester

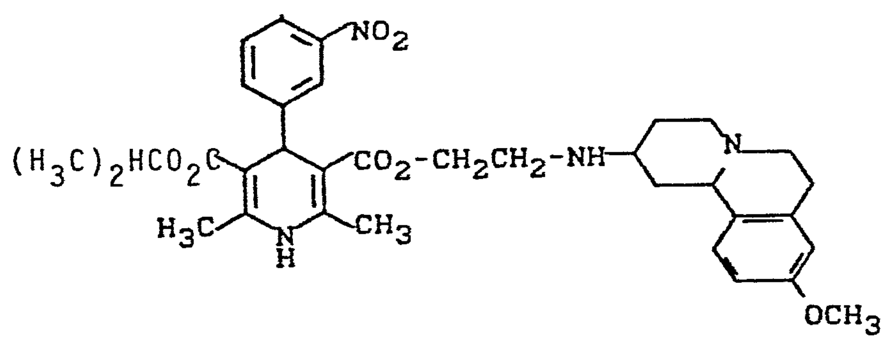

Ausbeute: 27,1 % der Theorie
$R_f$: 0,14 ($CHCl_3$:$CH_3OH$:$NH_3$/20:1:0,05)

Beispiel 10

1,4-Dihydro-2,6-dimethyl-4-(3-nitrophenyl)pyridin-3,5-dicarbonsäure-3-methylester-5-[6-(benzodioxan-2-ylmethylamino)hexyl]ester

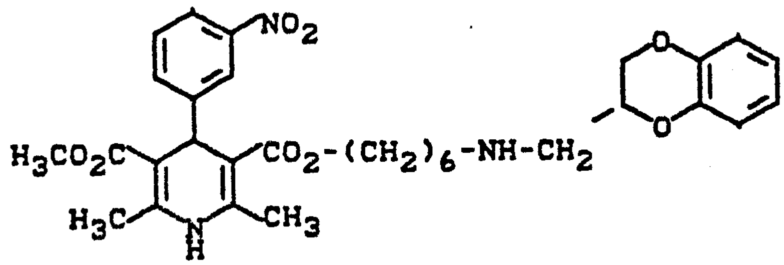

Verfahren A

430 mg (0,001 Mol) 1,4-Dihydro-2,6-dimethyl-4-(3-nitrophenyl)pyridin-3,5-dicarbonsäure-3-methylester-5-(6-aminohexyl)ester werden mit 230 mg (0,001 Mol) 2-Brommethyl-1,4-benzodioxan und 200 mg $K_2CO_3$ in 30 ml Dioxan p.a. 15 h am Rückfluß gekocht. Das Reaktionsgemisch wird abgekühlt und am Rotationsverdampfer eingeengt. Der Rückstand wird mit Natriumhydrogencarbonatlösung versetzt und 2 x mit je 50 ml Dichlormethan extrahiert. Die vereinigten organischen Phasen werden über Natriumsulfat getrocknet, filtriert und im Vakuum eingeengt. Den Rückstand reinigt man an einer Kieselgelsäule (Essigester : Methanol / 4:1). Das so erhaltene Produkt wird anschließend im Hochvakuum getrocknet.

Ausbeute: 310 mg (53% der Theorie)

Schmelzpunkt: amorph.

$R_f$: 0,43 (4:1/Essigester:$CH_3OH$)

Verfahren B

82 mg (0.0005 Mol) 2-Formyl-1,4-benzodioxan werden in 3 ml Methanol gelöst und mit methanolischer Salzsäure auf pH 6 gestellt. Dazu gibt man eine Lösung aus 200 mg (0,005 Mol) 1,4-Dihydro-2,6-dimethyl-4-(3-nitrophenyl)pyridin-3,5-dicarbonsäure-3-methylester-5-(6-aminohexyl)ester und 10 ml Methanol und verrührt das Reaktionsgemisch mit Molekularsieb 3Å 2,5 h bei Raumtemperatur. Anschließend werden portionsweise 32 mg Natriumcyanoborhydrid zugegeben und 20 h gerührt. Nach Filtration und Einengen am Rotationsverdampfer versetzt man den Rückstand mit Wasser und Methylenchlorid. Die organische Phase wird abgetrennt, mit Natriumsulfat getrocknet und eingeengt. Der Rückstand wird über eine Kieselgelsäule (Essigester) gereinigt. Das so erhaltene Produkt wird im Hochvakuum getrocknet.
Ausbeute: 158 mg (57% der Theorie)
Schmelzpunkt: amorph.
$R_f$: 0,49 (4:1/Essigester:$CH_3OH$)

Analog Beispiel 10 werden hergestellt:

Beispiel 11

1,4-Dihydro-2,6-dimethyl-4-(3-nitrophenyl)pyridin-3,5-dicarbonsäure-3-methylester-5-{2-[N-(1,4-benzodioxan-2-yl-methyl)-N-methylamino]ethyl}ester

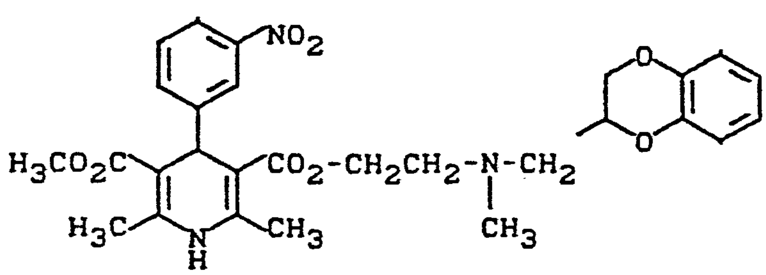

Schmelzpunkt: amorph.
Ausbeute: 21% der Theorie
$R_f$: 0,72 (Essigester)

Beispiel 12

1,4-Dihydro-2,6-dimethyl-4-(3-nitrophenyl)pyridin-3,5-dicarbonsäure-3-isopropylester-5-[2-(1,4-benzodioxan-2-yl-methylamino)ethyl]ester

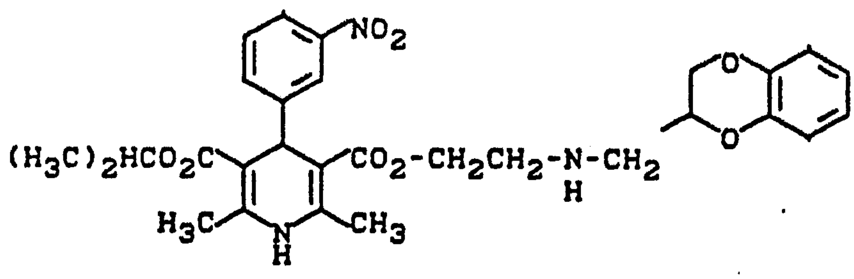

Schmelzpunkt: amorph
Ausbeute: 45% der Theorie
$R_f$: 0,41 (Essigester)

Beispiel 13

1,4-Dihydro-2,6-dimethyl-4-(3-nitrophenyl)pyridin-3,5-dicarbonsäure-3-methylester-5-[2-(1,4-benzodioxan-2-yl-methylamino)ethyl]ester

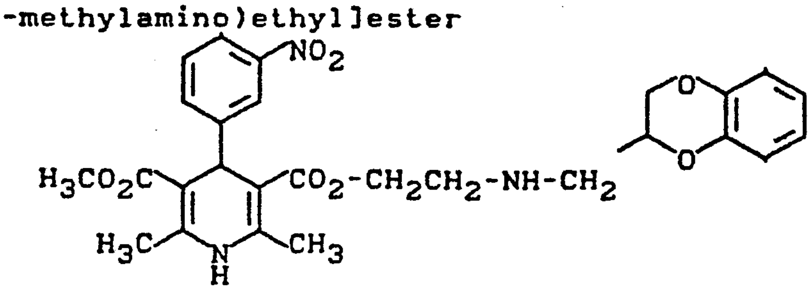

Ausbeute: 64% der Theorie
Schmelzpunkt: amorph.
$R_f$: 0,33 (Essigester)

Beispiel 14

1,4-Dihydro-2,6-dimethyl-4-(3-nitrophenyl)pyridin-3,5-dicarbonsäure-3-isopropyl-5-[3-(1,4-benzodioxan-2-yl-methylamino)propyl]ester

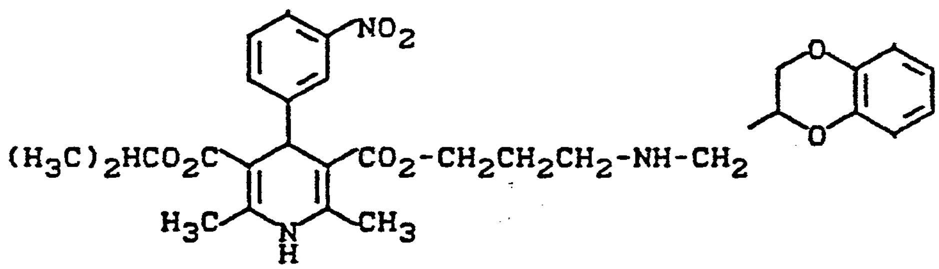

Ausbeute: 34% der Theorie
Schmelzpunkt: amorph.
$R_f$ : 0,72 (4:1/Essigester:$CH_3OH$)

Beispiel 15

1,4-Dihydro-2,6-dimethyl-4-(3-nitrophenyl)-pyridin-3,5-dicarbonsäure-3-methylester-5-{2-[2-(1,4-benzodioxan-2-yl-methyl)imidazol-1-yl]ethyl}ester

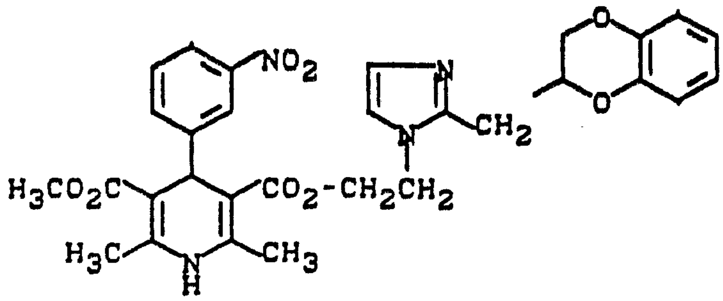

Unter Stickstoff werden 180 mg (0,006 Mol) Natriumhydroxid und 15 ml Dimethylformamid p.a. auf 0° C gekühlt und mit 620 mg (0,0025 Mol) versetzt. Man rührt 0,5 h bei Raumtemperatur und tropft anschließend eine Lösung aus 1,1 g (0,0025 Mol) 1,4-Dihydro-2,6-dimethyl-4-(3-nitrophenyl)pyridin-3,5-dicarbonsäure-3-methylester-5-(2-bromethyl)ester und 20 ml Dimethylformamid p.a. bei 0° C hinzu. Nach weiteren 30 Min. bei Raumtemperatur versetzt man das Reaktionsgemisch unter Kühlung tropfenweise mit $H_2O$ und extrahiert 3 mal mit Essigester. Die vereinigten organischen Phasen werden 3 mal mit $H_2O$ gewaschen, über Natriumsulfat getrocknet und am Rotavapor eingeengt. Der Rückstand wird über eine Kieselgelsäule (Essigester) gereinigt. Das so erhaltene Produkt wird im Hochvakuum getrocknet.

Ausbeute: 33% der Theorie

Schmelzpunkt: amorph.

$R_f$: 0,15 (Essigester)

Analog Beispiel 15 werden folgende Verbindungen hergestellt:

Beispiel 16

1,4-Dihydro-2,6-dimethyl-4-(3-nitrophenyl)pyridin-3,5-dicarbonsäure-3-methylester-5-{6-[2-(1,4-benzodioxan-2-yl-methyl)imidazol-1-yl]hexyl}ester

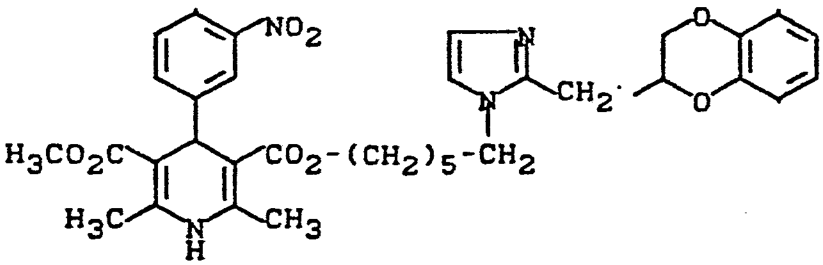

Ausbeute: 42% der Theorie
Schmelzpunkt: amorph.
$R_f$: 0,15 (Essigester)

Beispiel 17

1,4-Dihydro-2,6-dimethyl-4-(2-trifluormethylphenyl)pyridin-3,5-dicarbonsäure-3-methyl-5-{6-[2-(1,4-benzodioxan-2-yl-methyl)imidazol-1-yl]hexyl}ester

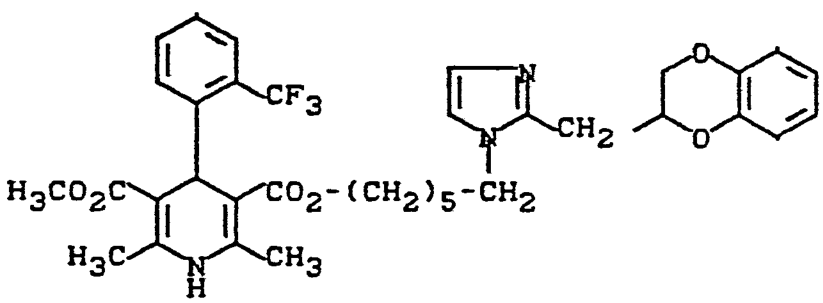

Ausbeute: 46% der Theorie
Schmelzpunkt: amorph.
$R_f$: 0,16 (Essigester)

Patentansprüche

1. Aminoester-Dihydropyridine der allgemeinen Formel (I)

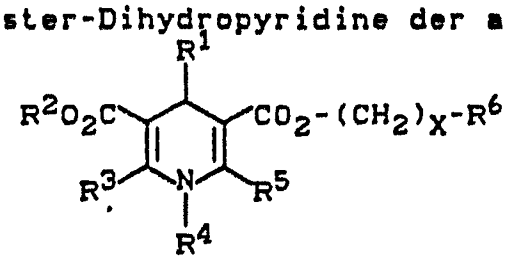

in welcher

$R^1$ - für $C_6$-$C_{14}$-Aryl oder für Heterocyclyl aus der Reihe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzothiadiazolyl, Chinolyl, Isochinolyl, Chinazolyl oder Chinoxalyl steht, wobei die genannten Ringsysteme jeweils durch ein oder zwei gleiche oder verschiedene Substituenten aus der Gruppe Phenyl, geradkettiges oder verzweigtes $C_1$-$C_4$-Alkyl, $C_3$-$C_7$-Cycloalkyl, $C_1$-$C_4$-Alkoxy, $C_1$-$C_4$-Alkylthio, Tri- oder Tetra- oder Pentamethylen, Dioxymethylen, Dioxyethylen, Halogen, Trifluormethyl, Trifluormethoxy, Difluormethoxy, Tetrafluorethoxy, Nitro, Cyano oder Azido substituiert sein können,

$R^2$ - für einen geradkettigen, verzweigten oder cyclischen, gesättigten oder ungesättigten Kohlenwasserstoffrest mit bis zu 15 Kohlenstoffatomen steht, der gegebenenfalls durch ein Sauerstoffatom oder ein Schwefelatom in der Kette unterbrochen ist und/oder der gegebenenfalls

substituiert ist durch Halogen, Cyano, Hydroxy, $C_2$-$C_7$-Acyloxy, Nitro, Nitrooxy oder durch eine Phenyl-, Phenoxy-, Phenylthio- oder Phenylsulfonylgruppe, die ihrerseits gegebenenfalls durch Di-$C_1$-$C_2$-alkylamino, $C_1$-$C_4$-Alkoxy, $C_1$-$C_4$-Alkyl, Halogen, Trifluormethyl oder Nitro substituiert ist, oder durch eine α-, β-oder γ-Pyridylgruppe, oder durch eine Aminogruppe, wobei diese Aminogruppe zwei gleiche oder verschiedene Substituenten aus der Gruppe $C_1$-$C_4$-Alkyl, $C_1$-$C_6$-Alkoxyalkyl, Phenyl und Benzyl trägt oder wobei die Substituenten gegebenenfalls mit dem Stickstoffatom einen 5-bis 7-gliedrigen Ring bilden, der als weiteres Heteroatom ein Sauerstoff-oder Schwefelatom oder eine N-Phenyl- oder N-Alkylgruppierung enthalten kann, wobei die Alkylgruppe ein bis drei Kohlenstoffatome umfaßt,

$R^3$ und $R^5$ gleich oder verschieden sind und jeweils

- für Wasserstoff, einen geradkettigen, verzweigten oder cyclischen Alkylrest mit bis zu 8 Kohlenstoffatomen, einen Phenyl- oder Benzylrest stehen,
  oder
  einer der Substituenten $R^3$ oder $R^5$ einen Alkylrest mit bis zu 6 Kohlenstoffatomen darstellt, der durch Acetoxy, Benzoyloxy, $C_1$-$C_3$-Alkoxy, $C_1$-$C_3$-Dialkoxy, Hydroxy, Amino, $C_1$-$C_6$-Aminoalkoxy, Phthalimido, $C_1$-$C_6$-Phthalimidoalkoxy, $C_1$-$C_6$-Piperidinoalkoxy, $C_1$-$C_6$-Morpholinoalkoxy oder N-Phenyl-N'-piperazinoalkoxy mit bis zu 6 Kohlenstoffatomen substituiert ist, oder
- für Formyl oder Nitril steht,

$R^4$ - für Wasserstoff oder

- für einen geradkettigen oder verzweigten Alkylrest mit bis zu vier Kohlenstoffatomen steht, der gegebenenfalls durch ein Sauerstoffatom in der Kette unterbrochen ist und/oder der gegebenenfalls durch einen Piperidino- oder Morpholino-Rest substituiert ist,

  oder

- für Phenyl oder Benzyl steht,

X - für eine Zahl von 2 bis 15 steht

und

$R^6$ - für einen Rest der Formel

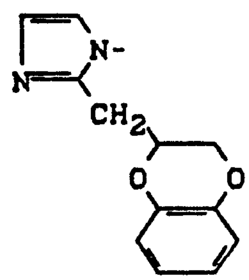

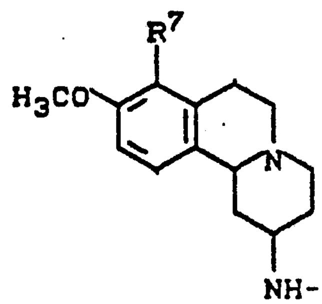

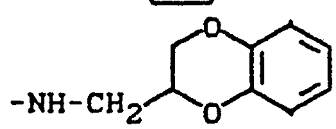

oder

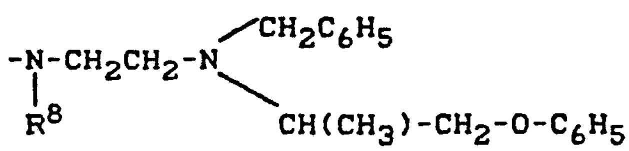

steht,

worin

$R^7$ -für Wasserstoff oder Methoxy steht und

$R^8$ -für Wasserstoff,
- für geradkettiges oder verzweigtes $C_1$-$C_6$-Alkyl oder
- für die Gruppe der Formel

$$-CH_2-CH_2-N\begin{array}{l} \diagup CH_2-C_6H_5 \\ \diagdown CH(CH_3)-CH_2-O-C_6H_5 \end{array}$$ steht,

und deren physiologisch unbedenklichen Salze.

2. Verbindungen der allgemeinen Formel (I) gemäß Anspruch 1, in welcher

$R^1$ - für Phenyl, Thienyl, Furyl, Pyridyl, Chinolyl oder Benzoxadiazolyl steht, wobei die genannten Ringsysteme gegebenenfalls durch ein bis zwei gleiche oder verschiedene Substituenten aus der Gruppe Phenyl, $C_1$-$C_2$-Alkyl, $C_1$-$C_2$-Alkoxy, $C_1$-$C_2$-Alkylthio, Dioxymethylen, Fluor, Chlor, Brom oder Iod, Trifluormethyl, Trifluormethoxy, Difluormethoxy, Nitro oder Cyano substituiert sind,

$R^2$ - für einen geradkettigen, verzweigten oder cyclischen, gesättigten oder ungesättigten Kohlenwasserstoffrest mit bis zu 10 Kohlenstoffatomen steht, der gegebenenfalls durch ein Sauerstoffatom in der Kette unterbrochen ist und/oder der

0236838

gegebenenfalls substituiert ist durch Fluor, Chlor, Brom, Iod, Cyano, Acetoxy, Hydroxy oder durch eine gegebenenfalls durch Fluor, Chlor, $C_1$-$C_2$-Alkyl, $C_1$-$C_2$-Alkoxy oder Trifluormethyl substituierte Phenyl- oder Phenoxygruppe, oder durch eine α-, β- oder γ-Pyridylgruppe, oder durch eine tertiäre Aminogruppe, wobei diese Aminogruppe zwei gleiche oder verschiedene Substituenten aus der Gruppe $C_1$-$C_4$-Alkyl, Phenyl oder Benzyl trägt,

$R^3$ und $R^5$ gleich oder verschieden sind und jeweils

- für einen geradkettigen, verzweigten oder cyclischen Alkylrest mit bis zu 6 Kohlenstoffatomen stehen,
  oder
  einer der Substituenten $R^3$ oder $R^5$ einen Alkylrest mit bis zu 4 Kohlenstoffatomen darstellt, der durch Acetoxy, Methoxy, Dimethoxy, Hydroxy, Amino, Phthalimido, Aminoalkoxy oder Phthalimidoalkoxy mit jeweils bis zu 4 Kohlenstoffatomen je Alkoxygruppe substituiert ist,
  oder
- für Formyl oder Cyano steht,

$R^4$ - für Wasserstoff oder
- für einen geradkettigen oder verzweigten Alkylrest mit bis zu vier Kohlenstoffatomen steht, der gegebenenfalls durch ein Sauerstoffatom in der Kette unterbrochen ist und/oder der gegebenenfalls durch einen Piperidino- oder Morpholino-Rest substituiert ist oder
- für Benzyl steht,

X - für eine Zahl von 2 bis 10 steht, und

$R^6$ - für einen Rest der Formel

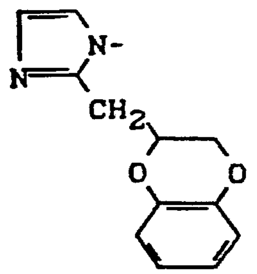

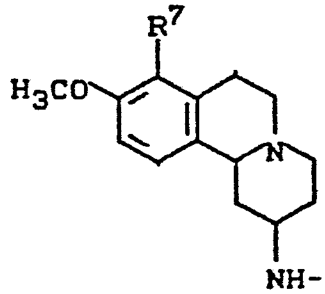

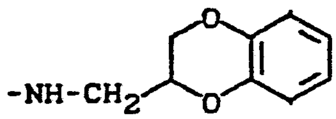

oder

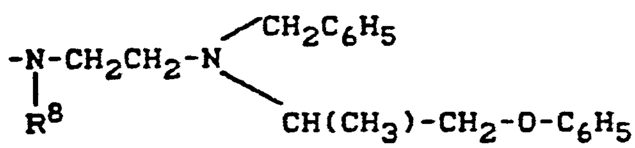

steht,

worin

$R^7$ - für Wasserstoff oder Methoxy steht und

$R^8$ - für Wasserstoff - für geradkettiges oder verzweigtes $C_1$-$C_4$-Alkyl
oder
- für den Rest der Formel

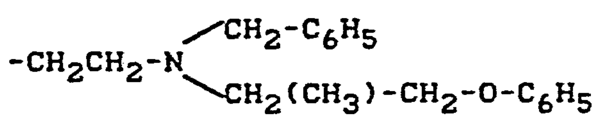

steht.

0236838

3. Verbindungen der allgemeinen Formel (I) gemäß Anspruch 1,

in welcher

$R^1$ - für Phenyl, Pyridyl oder Benzoxadiazolyl steht, wobei der Phenylring durch 1 oder 2 gleiche oder verschiedene Substituenten aus der Gruppe Chlor, Trifluormethyl, Nitro oder Cyano substituiert ist,

$R^2$ - für einen geradkettigen oder verzweigten Kohlenwasserstoffrest mit bis zu 7 Kohlenstoffatomen steht, der gegebenenfalls durch ein Sauerstoffatom in der Kette unterbrochen ist und/oder der gegebenenfalls substituiert ist durch Fluor, Cyano, Phenyl, α-, β- oder γ-Pyridyl oder durch N-Benzyl-N-methylamino,

$R^3$ und $R^5$ für Methyl stehen,

$R^4$ - für Wasserstoff steht,

X - für eine Zahl von 2 bis 6 steht,

und

$R^6$ - für einen Rest der Formel

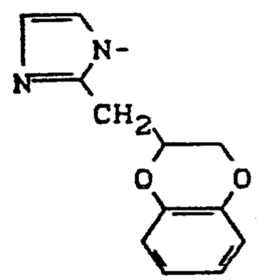

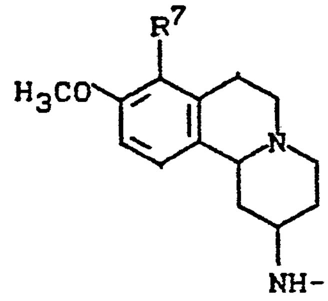

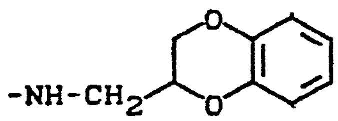

oder

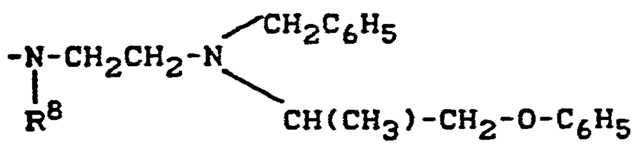

steht,

worin

$R^7$ - für Wasserstoff oder Methoxy steht und

$R^8$ - für Wasserstoff
- für geradkettiges oder verzweigtes $C_1$-$C_4$-Alkyl
oder
- für den Rest der Formel

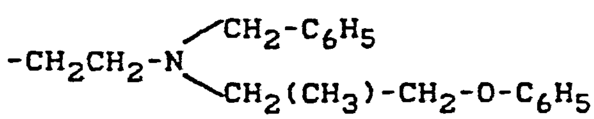

steht.

4. Verfahren zur Herstellung von Verbindungen der allgemeinen Formel (I)

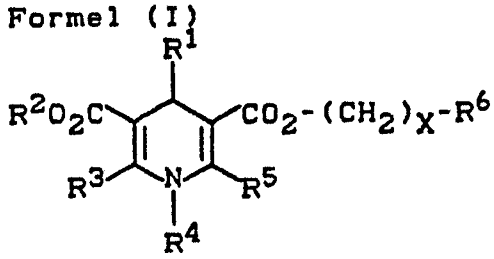

in welcher

$R^1$ - für $C_6$-$C_{14}$-Aryl oder für Heterocyclyl aus der Reihe Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzothiadiazolyl, Chinolyl, Isochinolyl, Chinazolyl oder Chinoxalyl steht, wobei die genannten Ringsysteme jeweils durch ein oder zwei gleiche oder verschiedene Substituenten aus der Gruppe Phenyl, geradkettiges oder verzweigtes $C_1$-$C_4$-Alkyl, $C_3$-$C_7$-Cycloalkyl, $C_1$-$C_4$-Alkoxy, $C_1$-$C_4$-Alkylthio, Tri- oder Tetra- oder Pentamethylen, Dioxymethylen, Dioxyethylen, Halogen, Trifluormethyl, Trifluormethoxy, Difluormethoxy, Tetrafluorethoxy, Nitro, Cyano oder Azido substituiert sein können,

$R^2$ - für einen geradkettigen, verzweigten oder cyclischen, gesättigten oder ungesättigten Kohlenwasserstoffrest mit bis zu 15 Kohlenstoffatomen steht, der gegebenenfalls durch ein Sauerstoffatom oder ein Schwefelatom in der Kette unterbrochen ist und/oder der gegebenenfalls

substituiert ist durch Halogen, Cyano, Hydroxy, $C_2$-$C_7$-Acyloxy, Nitro, Nitrooxy oder durch eine Phenyl-, Phenoxy-, Phenylthio- oder Phenylsulfonylgruppe, die ihrerseits gegebenenfalls durch Di-$C_1$-$C_2$-alkylamino, $C_1$-$C_4$-Alkoxy, $C_1$-$C_4$-Alkyl, Halogen, Trifluormethyl oder Nitro substituiert ist, oder durch eine α-, β-oder γ-Pyridylgruppe, oder durch eine Aminogruppe, wobei diese Aminogruppe zwei gleiche oder verschiedene Substituenten aus der Gruppe $C_1$-$C_4$-Alkyl, $C_1$-$C_6$-Alkoxyalkyl, Phenyl und Benzyl trägt oder wobei die Substituenten gegebenenfalls mit dem Stickstoffatom einen 5-bis 7-gliedrigen Ring bilden, der als weiteres Heteroatom ein Sauerstoff-oder Schwefelatom oder eine N-Phenyl- oder N-Alkylgruppierung enthalten kann, wobei die Alkylgruppe ein bis drei Kohlenstoffatome umfaßt,

$R^3$ und $R^5$ gleich oder verschieden sind und jeweils

- für Wasserstoff, einen geradkettigen, verzweigten oder cyclischen Alkylrest mit bis zu 8 Kohlenstoffatomen, einen Phenyl- oder Benzylrest stehen,

oder

einer der Substituenten $R^3$ oder $R^5$ einen Alkylrest mit bis zu 6 Kohlenstoffatomen darstellt, der durch Acetoxy, Benzoyloxy, $C_1$-$C_3$-Alkoxy, $C_1$-$C_3$-Dialkoxy, Hydroxy, Amino, $C_1$-$C_6$-Aminoalkoxy, Phthalimido, $C_1$-$C_6$-Phthalimidoalkoxy, $C_1$-$C_6$-Piperidinoalkoxy, $C_1$-$C_6$-Morpholinoalkoxy oder N-Phenyl-N'-piperazinoalkoxy mit bis zu

6 Kohlenstoffatomen substituiert ist, oder
- für Formyl oder Nitril steht,

$R^4$ - für Wasserstoff oder
- für einen geradkettigen oder verzweigten Alkylrest mit bis zu vier Kohlenstoffatomen steht, der gegebenenfalls durch ein Sauerstoffatom in der Kette unterbrochen ist und/oder der gegebenenfalls durch einen Piperidino- oder Morpholino-Rest substituiert ist,
oder
- für Phenyl oder Benzyl steht,

X - für eine Zahl von 2 bis 15 steht

und

$R^6$ - für einen Rest der Formel

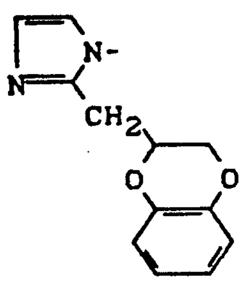

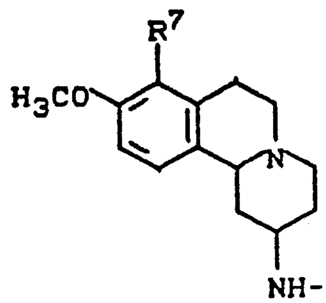

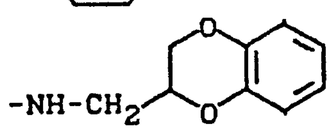

oder

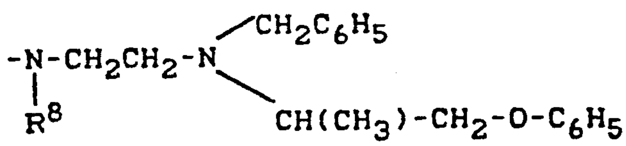

steht,

worin

$R^7$ -für Wasserstoff oder Methoxy steht und

$R^8$ -für Wasserstoff,
- für geradkettiges oder verzweigtes $C_1$-$C_6$-Alkyl oder
- für die Gruppe der Formel

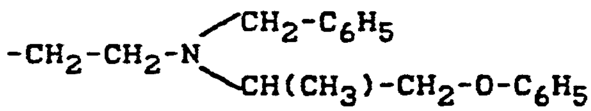

steht,

dadurch gekennzeichnet, daß man Verbindungen der allgemeinen Formel (II)

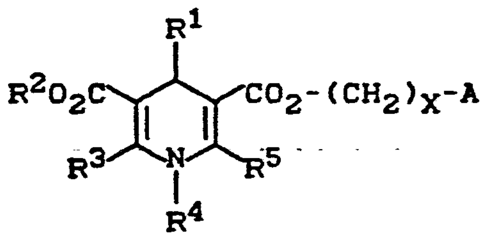

(II)

in welcher

$R^1$-$R^5$ und X die oben angegebene Bedeutung haben und

A für den Rest $NH^8$ steht, wobei $R^8$ die oben angegebene Bedeutung hat, oder für Halogen steht,

0236838

– mit Verbindungen der Formel (III)

$$B\text{-}R^6 \qquad \text{(III)}$$

in welcher

$R^6$ die oben angegebene Bedeutung hat und

B für einen reaktiven Rest aus der Gruppe Halogen, Carbonyl oder eine sekundäre Aminogruppe steht,

in inerten organischen Lösungsmitteln bei Temperaturen zwischen 0 bis 200°C umsetzt und bei der Verwendung von Ausgangsverbindungen der Formel (III) mit reaktiven Carbonylgruppen die erhaltenen Schiff'schen Basen nach üblichen Methoden reduziert.

5. Verfahren zur Herstellung von Verbindungen der allgemeinen Formel (Ia)

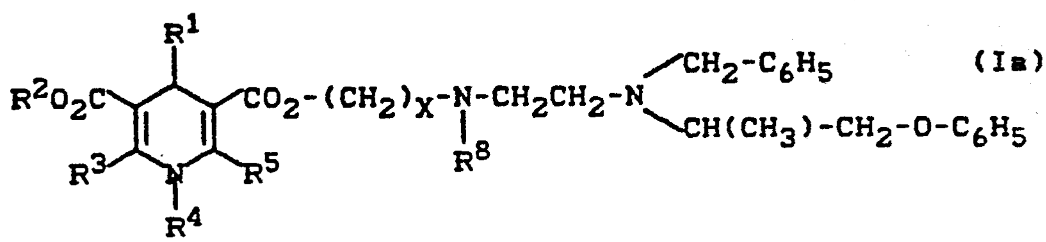

(Ia)

in welcher

$R^1$-$R^5$,$R^8$ und X die in Anspruch 4 angegebene Bedeutung haben,

dadurch gekennzeichnet, daß man Amino-Verbindungen der allgemeinen Formel (IIa)

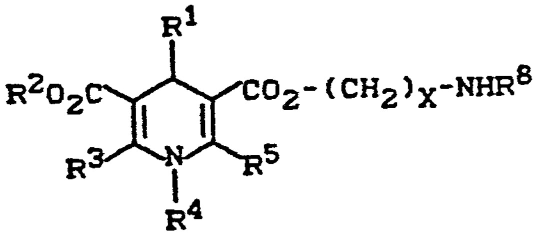

(IIa)

in welcher
$R^1$-$R^5$, X und $R^8$ die angegebene Bedeutung haben,

mit Halogenverbindungen der allgemeinen Formel (IIIa)

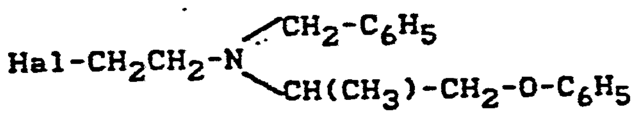

(IIIa)

in welcher
Hal - für Fluor, Chlor, Brom oder Iod, bevorzugt für Chlor oder Brom steht,

in inerten organischen Lösungsmitteln, gegebenenfalls in Anwesenheit einer Base umsetzt, gegebenenfalls anschließend alkyliert, und gegebenenfalls mit Säuren die physiologisch unbedenklichen Salze herstellt.

6. Verfahren zur Herstellung von Verbindungen der allgemeinen Formel (Ib)

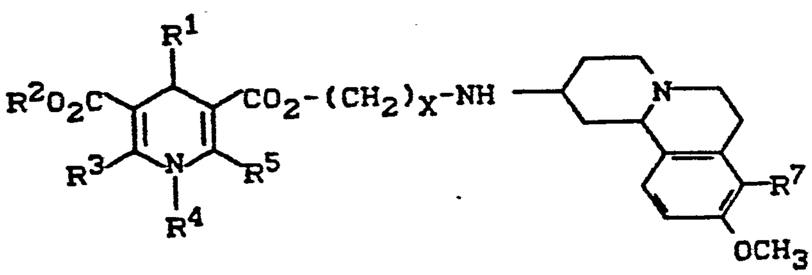

(Ib),

0236838

in welcher
$R^1$-$R^5$, $R^7$ und X die in Anspruch 4 angegebene Bedeutung haben,

dadurch gekennzeichnet, daß man in einem ersten Schritt Ketone der Formel (IIIb)

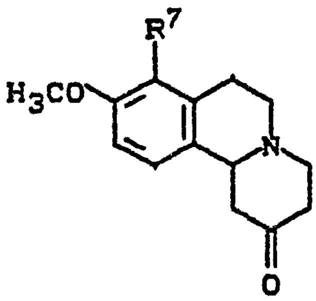

(IIIb)

mit Aminen der Formel (IIa)
in welchen
$R^1$-$R^5$, $R^7$ und X die angegebene Bedeutung haben,
und
$R^8$ - für Wasserstoff steht,

in einem inerten organischen Lösungsmittel gegebenenfalls in Gegenwart eines Katalysators umsetzt, die so erhaltenen Schiffschen Basen in einem zweiten Schritt in inerten Lösungsmitteln reduziert und gegebenenfalls anschließend mit Säuren die pharmakologisch unbedenklichen Salze herstellt.

7. Verfahren zur Herstellung von Verbindungen der allgemeinen Formel (Ic)

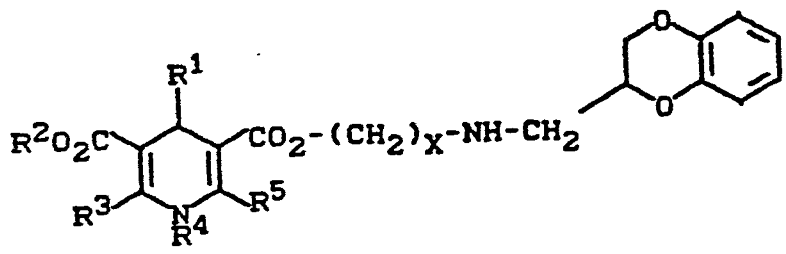

(Ic)

in welcher

$R^1$-$R^5$ und X die in Anspruch 4 angegebene Bedeutung haben,

dadurch gekennzeichnet, daß man [1] Amino-Dihydropyridine der allgemeinen Formel (IIa)

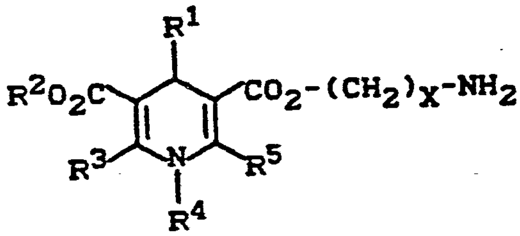

(IIa)

in welcher

$R^1$-$R^5$ und X die angegebene Bedeutung haben,

mit Halogenverbindungen der Formel (IIIc)

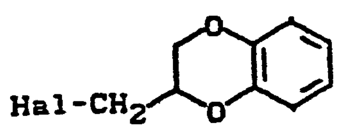

(IIIc)

in welcher
Hal- für Fluor, Chlor, Brom oder Iod,
bevorzugt für Chlor oder Brom steht,

in inerten organischen Lösungsmitteln, gegebennfalls in Anwesenheit einer Base umsetzt, und gegebenenfalls mit Säuren die physiologisch unbedenklichen Salze herstellt,

oder indem man

[2] Amino-Dihydropyridine der Formel (IIa) mit Carbonylverbindungen der Formel (IIIcc)

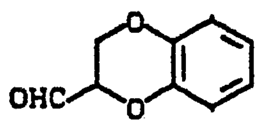

(IIIcc)

in einem inerten Lösungsmittel gegebenenfalls in Gegenwart eines Katalysators umsetzt, die so erhaltenen Schiffschen Basen in einem zweiten Schritt in inerten Lösungsmitteln reduziert und gegebenenfalls anschließend mit Säuren die physiologisch unbedenklichen Salze herstellt.

8. Verfahren zur Herstellung von Verbindungen der allgemeinen Formel (Id)

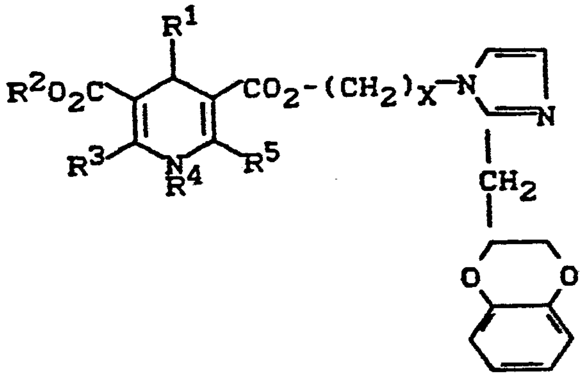

(Id)

in welcher $R^1$-$R^5$ und X die in Anspruch 4 angegebene Bedeutung haben,

dadurch gekennzeichnet, daß man Dihydropyridinhalogenester der allgemeinen Formel (IId)

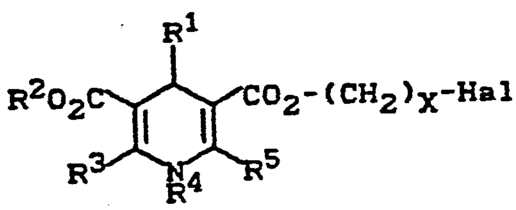

(IId)

in welcher

$R^1$-$R^5$ und X die angegebene Bedeutung haben und

Hal - für Fluor, Chlor, Brom, Iod, bevorzugt für Chlor oder Brom steht,

mit dem Imidazol der Formel (IIId)

0236838

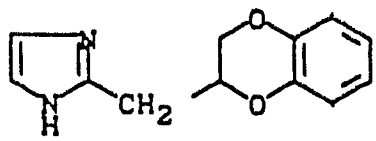

(IIId)

in inerten organischen Lösungsmitteln, gegebenfalls in Anwesenheit einer Base umsetzt.

9. Verbindungen gemäß Ansprüchen 1 - 3 zur Bekämpfung von Erkrankungen.

10. Verwendung der Verbindungen gemäß Ansprüchen 1 - 3 zur Herstellung von Arzneimitteln zur Behandlung ischämischer Herzerkrankungen und essentiellem Hochdruck.

11. Arzneimittel, enthaltend als Wirkstoff eine Verbindung gemäß Ansprüchen 1 - 3.

12. Verfahren zur Herstellung von Arzneimitteln, dadurch gekennzeichnet, daß man Verbindungen der allgemeinen Formel I gemäß Ansprüchen 1 und 4 gegebenenfalls unter Verwendung von Hilfs- und Trägerstoffen in eine geeignete Applikationsform überführt.